(19) **Europäisches Patentamt**
**European Patent Office**
**Office européen des brevets**

(11) **EP 3 239 377 B1**

(12) # EUROPEAN PATENT SPECIFICATION

(45) Date of publication and mention
of the grant of the patent:
**24.11.2021 Bulletin 2021/47**

(51) Int Cl.:
***D04H 1/4391*** *(2012.01)*  ***D04H 1/492*** *(2012.01)*
***D04H 1/541*** *(2012.01)*  ***A61F 13/02*** *(2006.01)*

(21) Application number: **15873363.4**

(22) Date of filing: **25.12.2015**

(86) International application number:
**PCT/JP2015/086422**

(87) International publication number:
**WO 2016/104795 (30.06.2016 Gazette 2016/26)**

(54) **SELF-ADHERING NONWOVEN FABRIC**

SELBSTHAFTENDER VLIESSTOFF

TISSU NON-TISSÉ AUTO-ADHÉSIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.12.2014 JP 2014265434**

(43) Date of publication of application:
**01.11.2017 Bulletin 2017/44**

(73) Proprietor: **Kuraray Co., Ltd.
Okayama 710-0801 (JP)**

(72) Inventors:
• **KOIZUMI, Satoshi
Okayama-shi
Okayama 702-8045 (JP)**

• **KIYOOKA, Sumito
Okayama-shi
Okayama 702-8045 (JP)**

(74) Representative: **Vossius & Partner
Patentanwälte Rechtsanwälte mbB
Siebertstrasse 3
81675 München (DE)**

(56) References cited:
**EP-A1- 2 058 424     EP-A1- 2 371 331
WO-A1-2008/015972   WO-A1-2012/070556
WO-A1-2014/033417   JP-A- 2009 183 363
JP-A- 2009 184 697   JP-A- 2012 012 758
JP-A- 2014 037 662   JP-A- 2014 037 662
JP-A- 2015 047 816**

• **None**

**Description**

TECHNICAL FIELD

The present invention relates to a self-adhering nonwoven fabric that can be suitably used as a bandage or the like.

BACKGROUND ART

Bandages are used for directly protecting an application part such as an affected area by being wound around the application part, or for fixing other protecting member (such as gauze) to an application part. Besides these uses, a bandage having stretchability is also used for stopping bleeding or promoting the blood flow to alleviate the edema in the wound part by the pressurizing force at the time of winding with the help of the stretchability. In recent years, bandages have been also applied to the pressure therapy in which the treatment is made by pressurizing the affected area, such as the treatment or the amelioration of a varicose vein of the lower extremities. As a stretchable bandage, a nonwoven fabric or the like is used.

A bandage needs to be fixed at its leading end after it is wound around an application part or the like. As the fixing means, for example, a method of applying an adhesive on the surface of the bandage, and a method of separately preparing a stopper for fixing the leading end of the bandage have been conventionally known. However, the former method has a problem that the adhesive can cause skin irritation or induce allergy, and the latter method has a problem that handling of the bandage is complicated.

As a bandage capable of solving the above problems, a self-adhering bandage made of a nonwoven fabric has been proposed [International Publication WO 2008/015972 A (PTD 1)]. A self-adhering bandage refers to a bandage having such a characteristic that bandage parts (for example, the leading end part after winding the bandage around an application part or the like, and the wound part of the bandage beneath the leading end part) are engaged and fixed with each other by overlapping these bandage parts without using an adhesive, a stopper or the like. A self-adhering bandage as described in PTD 1 has hand-cuttability, or in other words, it can be broken (cut) by stretching with a hand without using cutting means such as scissors. PTD 2 similarly discloses a nonwoven fabric that can be easily torn by hand, which comprises conjugated fibers forming specific crimps and entangled with each other. PTD 3 further discloses a high stress sheet that is elastic, self-adhesive and hand-tearable.

CITATION LIST

PATENT DOCUMENT

   PTD 1: International Publication WO 2008/015972 A

   PTD 2: EP 2 058 424 A1

   PTD 3: JP 2014 037662 A

SUMMARY OF INVENTION

The invention for which protection is sought is defined by the independent claim(s). The dependent claims concern particular embodiments. Any disclosure that does not fall within the claims should be deemed as being presented for illustrative purpose.

TECHNICAL PROBLEMS

A self-adhering bandage is often used in an environment exposed to the external force as in the case of being wound around a joint part, and once it is wound around an application part or the like, it tends to be continuously used for a long term in that condition. Therefore, excellent self-adhesiveness is required for the self-adhering bandage, and excellent self-adhesiveness is required, in particular, for the leading end part formed by breakage by stretching typified by hand-cutting (cutting with a hand). This is because if the self-adhesiveness of the leading end part is insufficient, the leading end part would become easier to peel up due to the external force or the long-term use, and the adhesion of the remaining part would gradually come loose from that part, and finally the bandage would come off.

In light of the above, it is an object of the present invention to provide a nonwoven fabric in which self-adhesiveness of a leading end part formed by breakage by stretching is improved, and a bandage using the same.

SOLUTIONS TO PROBLEMS

The present invention provides the following nonwoven fabrics.

[1] A nonwoven fabric, wherein in a broken end part formed by a tensile test in accordance with JIS L 1913 of stretching in one direction to lead breakage, letting an innermost point in said one direction be $P_{in}$ and an outermost point be $P_{out}$, distance D along said one direction between point $P_{in}$ and point $P_{out}$ is less than or equal to 50 mm, as defined in claim 1.

Further nonwoven fabrics are defined in dependent claims 2-13.

ADVANTAGEOUS EFFECTS OF INVENTION

According to the present invention, it is possible to provide a self-adhering nonwoven fabric capable of being broken (cut) by stretching, and forming a leading end part having excellent self-adhesiveness by the breakage. The nonwoven fabric according to the present invention is suited for a bandage.

BRIEF DESCRIPTION OF DRAWINGS

Fig. 1 is a view for illustrating distance D (broken end part length), and is a schematic top view showing one example of a broken end part that is formed when a nonwoven fabric is broken by stretching.
Fig. 2 is a schematic top view of a nonwoven fabric for illustrating a rectangular area and divisional areas contained in the same.
Fig. 3 is a schematic view showing a method for preparing a sample for measuring curved surface sliding stress.
Fig. 4 is a sectional schematic view showing a sample for measuring curved surface sliding stress.
Fig. 5 is a schematic view showing a method for measuring curved surface sliding stress.
Fig. 6 is a schematic view showing a method for measuring curvature of fiber.

DESCRIPTION OF EMBODIMENTS

<Nonwoven fabric>

(1) Characteristics of nonwoven fabric

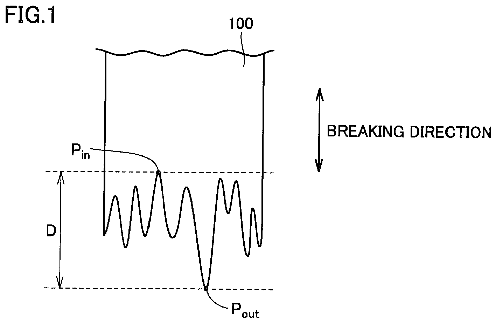

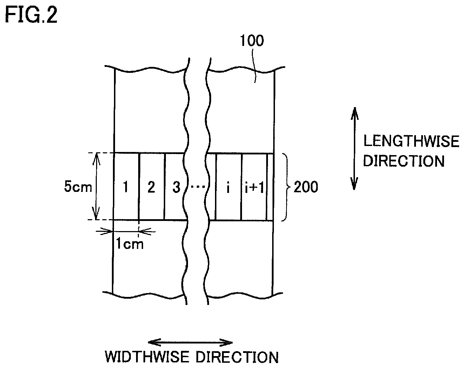

The nonwoven fabric according to the present invention is a nonwoven fabric having such a property that by overlapping (contacting) fibers on surfaces of the nonwoven fabric, they are engaged with each other and fixed, namely a nonwoven fabric having self-adhesiveness. In particular, the present invention focuses on the self-adhesiveness in the leading end part (hereinafter, also referred to as "broken end part") of the nonwoven fabric that is formed by breakage by stretching as in the case of breaking (cutting) a nonwoven fabric by stretching with a hand, and one feature of the nonwoven fabric according to the present invention lies in this broken end part. The feature of the broken end part will be described by referring to Fig. 1 and Fig. 2.

Fig. 1 illustrates one example of a broken end part that is formed when a nonwoven fabric 100 is broken by stretching. As shown in Fig. 1, in general, when nonwoven fabric 100 is broken by stretching, a cilium extending in the stretching direction [a fiber constituting the nonwoven fabric or an aggregate (bundle) thereof] is exposed in a broken end part (cut section) thereof. While such a cilium can arise plurally in the broken end part, normally, the projecting (extending) lengths thereof are different from each other, and distribution occurs. Referring to Fig. 1, in the nonwoven fabric according to the present invention, in a broken end part formed by breakage by stretching in one direction, letting an innermost point in the one direction be $P_{in}$ and an outermost point be $P_{out}$, distance D along the one direction between point $P_{in}$ and point $P_{out}$ (hereinafter, referred to as "broken end part length") is less than or equal to 50 mm. The one direction is a breaking direction when the nonwoven fabric is broken by stretching, and is synonymous to the stretching direction.

In the nonwoven fabric of the present invention capable of forming a broken end part having broken end part length D of less than or equal to 50 mm, since the broken end part (cut section) is relatively even along the direction orthogonal to the breaking direction, the self-adhesiveness in the broken end part is excellent. In other words, even when the nonwoven fabric is self-adhered in such an environment that it is exposed to external forces from various directions, or when the nonwoven fabric is self-adhered for a long term, the self-adhered broken end part is less likely to be peeled off. According to the nonwoven fabric according to the present invention, it is possible to improve the peeling resistance against the external force directed from the outer side to the inner side of the nonwoven fabric, namely the external force in the breaking direction, and it is possible to improve the peeling resistance against the external forces from other directions such as a direction orthogonal to the breaking direction (external force applied laterally). When broken end part length D exceeds 50 mm, the self-adhesiveness of the broken end part significantly deteriorates. Since the nonwoven fabric according to the present invention has excellent self-adhesiveness by itself, it is preferred not to substantially contain an adhesive.

The nonwoven fabric according to the present invention preferably has two or more sites in which a broken end part having broken end part length D of less than or equal to 50 mm can be formed, and more preferably has three or more such sites, and further preferably shows broken end part length D of less than or equal to 50 mm regardless of the position in the breaking direction where it is broken.

From the view point of further improvement in the self-adhesiveness of the broken end part, broken end part length D is preferably less than or equal to 45 mm, more preferably less than or equal to 40 mm. On the other hand, when broken end part length D is 0 mm (as if it were cut with scissors), the self-adhesiveness of the broken end part tends to be poor, and hence broken end part length D is preferably greater than or equal to 0.1 mm, more preferably greater than or equal to 0.5 mm, further preferably greater than or equal to 1 mm.

The nonwoven fabric according to the present invention is typically a nonwoven fabric having hand-cuttability, and typically aims at improving the self-adhesiveness of the broken end part generated by hand cutting (cutting with a hand). Therefore, the nonwoven fabric according to the present invention can be provided with a cutting-facilitating mechanism such as cutting lines, for example, by making intermittent cuts along a predetermined line so that cutting along the line is facilitated, however, broken end part length D according to the present invention does not basically involve the broken end part length when cutting is performed with such a cutting-facilitating mechanism. In other words, when the nonwoven fabric according to the present invention has a cutting-facilitating mechanism, the part that is broken for measuring broken end part length D is other part than the cutting-facilitating mechanism.

Broken end part length D is measured by breaking a nonwoven fabric by stretching in the aforementioned one direction to generate a broken end part. For this purpose, the tensile test in accordance with JIS L 1913 "Test methods for nonwovens" is used. When the breaking strength of the nonwoven fabric is measured together by the tensile test in accordance with JIS L 1913 "Test methods for nonwovens", the breakage sample obtained by the measurement can be used as a sample for measuring broken end part length D. For each of the two broken end parts of the breakage sample obtained by the aforementioned tensile test, innermost point $P_{in}$ and outermost point $P_{out}$ are specified, and a distance from point $P_{in}$ to point $P_{out}$ along the breaking direction (stretching direction) is determined, and a mean value of the two distances determined for the two breakage samples is regarded broken end part length D. In specifying point $P_{out}$, when cilia exposed in the broken end part include a cilium that extends in the direction nonparallel with breaking direction, point $P_{out}$ is specified while the orientation is adjusted so that the direction in which the cilium extends is parallel with the breaking direction with the care of not causing the variation of the projecting (extending) length itself.

The shape of the nonwoven fabric according to the present invention is normally a sheet shape, and more specific shapes can be selected depending on the use application, however, rectangular sheets having a lengthwise direction and a widthwise direction, such as a tape shape or a band shape (long) shape are preferred. In this case, it is preferred that the nonwoven fabric according to the present invention forms a broken end part having broken end part length D of less than or equal to 50 mm when at least the breaking direction is parallel with the lengthwise direction. The nonwoven fabric according to the present invention preferably has two or more sites in which a broken end part having broken end part length D of less than or equal to 50 mm when at least the breaking direction is parallel with the lengthwise direction can be formed, and more preferably has three or more such sites, and particularly preferably shows broken end part length D of less than or equal to 50 mm regardless of the position in the breaking direction (lengthwise direction) where it is broken. The lengthwise direction of the nonwoven fabric can be a machine direction in the manufacturing process (MD direction), and the widthwise direction of the nonwoven fabric can be a direction orthogonal to the machine direction of the nonwoven fabric in the manufacturing process (CD direction).

The nonwoven fabric according to the present invention having a lengthwise direction and a widthwise direction preferably contains a specific rectangular area 200 having a length in the lengthwise direction of 5 cm, a length in the widthwise direction equivalent to the overall width of the nonwoven fabric. Referring to Fig. 2, specific rectangular area 200 refers to a rectangular area wherein in two or more divisional areas contained in the rectangular area each having a length in the lengthwise direction of 5 cm and a length in the widthwise direction of 1 cm, a ratio $W_i/W_{i+1}$ between a mass per unit area $W_i$ of an i-th divisional area from one end of the widthwise direction of the nonwoven fabric (provided that i is an integer of greater than or equal to 1, and a first divisional area contains the one end of the widthwise direction) and a mass per unit area $W_{i+1}$ of an (i+1)th divisional area is 0.9 to 1.1. According to the nonwoven fabric containing such rectangular area 200, broken end part length D of less than or equal to 50 mm can be exhibited by breaking the nonwoven fabric by stretching in such a manner that the breakage occurs in rectangular area 200. Nonwoven fabric 100 containing specific rectangular area 200 has a length in the lengthwise direction of at least 5 cm, and a length in the widthwise direction of at least 2 cm.

While rectangular area 200 is required to satisfy the relation $W_i/W_{i+1}$ = 0.9 to 1.1 for at least one i, the relation is satisfied preferably for two or more "i"s (when three or more divisional areas are contained), more preferably for three or more "i"s (when four or more divisional areas are contained), further preferably for all "i"s from the view point of further improvement of the self-adhesiveness of the broken end part; and it is particularly preferred that the ratio between the mass per unit area $W_i$ of said i-th divisional area and the mass per unit area of each of other divisional areas respectively satisfies the relation (in the case of containing three or more divisional areas), and it is most preferred that the ratio between the mass per unit area $W_i$ of said i-th divisional area and the mass per unit area of each of other divisional areas respectively satisfies the relation (in the case of containing three or more divisional areas) for all "i"s.

As described above, the first divisional area is assigned to include one end in the widthwise direction of nonwoven fabric 100 (see Fig. 2). At this time, in the other end in the widthwise direction in rectangular area 200, a margin area of 1 cm wide for which a divisional area cannot be assigned may be formed. In other words, the overall width of the nonwoven fabric according to the present invention is not limited to integral multiples of 1 cm.

The nonwoven fabric according to the present invention preferably has two or more specific rectangular areas 200, more preferably three or more specific rectangular areas 200, and it is further preferred that the substantially entire area or almost the entire area of the nonwoven fabric is configured by specific rectangular area 200. When the nonwoven fabric has two or more specific rectangular areas 200, these rectangular areas 200 may be continuous, or may be distanced from each other.

The density (bulk density) of the nonwoven fabric according to the present invention is for example, 0.03 to 0.5 $g/cm^3$, preferably 0.05 to 0.2 $g/cm^3$, more preferably 0.06 to 0.18 $g/cm^3$. When the density of the nonwoven fabric falls within this range, fibers are more likely to come into engagement with each other, and more excellent self-adhesiveness is realized. The thickness of the nonwoven fabric is, for example, 0.2 to 5 mm, preferably 0.3 to 3 mm, more preferably 0.4 to 2 mm. The mass per unit area as a whole of the nonwoven fabric, namely, the mean mass per unit area of the nonwoven fabric is preferably greater than or equal to 30 $g/m^2$, more preferably greater than or equal to 50 $g/m^2$. When the mass per unit and the thickness fall within these ranges, the balance between the stretchability and the flexibility of the nonwoven fabric, and the balance between the drape and the cushioning characteristics of the nonwoven fabric are excellent.

The nonwoven fabric according to the present invention includes a plurality of low-density parts and a plurality of high-density parts that are arranged along a certain direction in the plane (for example, lengthwise direction), and the low-density part and the high-density part are arranged alternately and periodically. By providing difference in density in the plane of the nonwoven fabric, it is possible to improve the stretchability while ensuring the excellent hand-cuttability. The arrangement pattern of the low-density parts and the high-density parts is not particularly limited, but when the nonwoven fabric has a lengthwise direction and a widthwise direction, it may be a striped pattern in which the low-density part and the high-density part are alternately arranged along the lengthwise direction, or a pattern in which they are arranged alternately in a mesh pattern or a lattice (staggered) pattern. A mesh pattern or a lattice (staggered) pattern is preferred. In the mesh or lattice pattern, the areal percentages of the low-density parts and the high-density parts may be different or the same with each other. The areal ratio of low-density part/high-density part is, for example, 90/10 to 10/90, preferably 70/30 to 30/70. The mean widths of the low-density parts and the high-density parts are, for example, 0.1 to 10 mm, preferably 0.5 to 5 mm, more preferably 1 to 3 mm.

The compressive stress of the nonwoven fabric according to the present invention is preferably 0.2 to 10 kPa, more preferably 0.5 to 5 kPa, further preferably 0.6 to 4 kPa. When the compressive stress of the nonwoven fabric falls within this range, the nonwoven fabric is imparted with appropriate cushioning characteristics, and the integrity when the fibers are brought into contact with each other is improved, and thus the self-adhesiveness of the nonwoven fabric, in particular, the self-adhesiveness of the broken end part can be improved. The compressive stress of the nonwoven fabric is measured according to the method described in the section of Examples.

In the nonwoven fabric according to the present invention, the breaking strength for at least one direction in the plane direction is for example, 5 to 30 N/50 mm, preferably 6 to 25 N/50 mm, more preferably 7 to 20 N/50 mm. The breaking strength is related with the hand-cuttability of the nonwoven fabric. The nonwoven fabric according to the present invention preferably has excellent hand-cuttability that allows relatively easy hand breaking (cutting). If the breaking strength is too large, the hand-cuttability is insufficient, so that it becomes difficult to cut the nonwoven fabric, for example, with only one hand. If the breaking strength is too small, the strength of the nonwoven fabric is insufficient, and the nonwoven fabric is easily broken, so that the handleability deteriorates. The breaking strength is measured by a tensile test in accordance with JIS L 1913 "Test methods for nonwovens".

The aforementioned "at least one direction in the plane direction" can be a machine direction of the nonwoven fabric in the manufacturing process (MD direction), and when the nonwoven fabric has a lengthwise direction and a widthwise direction as in the case of a bandage, for example, "at least one direction in the plane direction" is preferably the lengthwise direction of the nonwoven fabric. That is, when the nonwoven fabric is used as a bandage, a certain degree of strength is required for winding a required amount of the bandage around an affected area while stretching the bandage along its lengthwise direction and then breaking the bandage to fix the broken end part. Therefore, it is preferred that the nonwoven fabric according to the present invention satisfies the aforementioned range of the breaking strength in the lengthwise direction.

In production of a bandage with the use of the nonwoven fabric according to the present invention, it is necessary to work the nonwoven fabric in accordance with the width and the length required for the bandage, and normally, this process can be easily conducted by using a slitter rewinder. Therefore, also from the view point of ensuring the excellent productivity, the breaking strength preferably falls within the above range in the lengthwise direction of the nonwoven fabric.

On the other hand, the breaking strength can be relatively small in other directions than the aforementioned at least one direction in the plane direction, for example in the direction orthogonal to the machine direction of the manufacturing process (CD direction), or in the widthwise direction when the nonwoven fabric has the lengthwise direction and the widthwise direction as in the case of a bandage, and is for example, 0.05 to 20 N/50 mm, preferably 0.1 to 15 N/50 mm, more preferably 0.5 to 10 N/50 mm.

As described above, the nonwoven fabric according to the present invention normally has anisotropy between the MD direction and the CD direction. That is, in the manufacturing process, the nonwoven fabric of the present invention has such a tendency that not only the axial direction of the coiled crimped fibers becomes substantially parallel with the plane direction, but also the axial direction of the coiled crimped fibers oriented substantially parallel with the plane direction becomes substantially parallel with the MD direction. As a result, in production of a nonwoven fabric having a lengthwise direction and a widthwise direction, stretching characteristics and breaking characteristics, in particular, the breaking strength have anisotropy between the MD direction and the CD direction. When the nonwoven fabric is used as a bandage, by assigning the MD direction for the lengthwise direction of nonwoven fabric, it is possible to obtain a nonwoven fabric having a breaking strength in the lengthwise direction falling within the above range. Regarding the anisotropy of the breaking strength, the breaking strength in the lengthwise direction (MD direction) of the nonwoven fabric is for example, 1.5 to 50 times, preferably 2 to 40 times, more preferably 3 to 30 times, relative to the breaking strength in the widthwise direction.

In the nonwoven fabric according to the present invention, the breaking elongation in at least one direction in the plane direction is for example, greater than or equal to 50%, preferably greater than or equal to 60%, more preferably greater than or equal to 80%. The breaking elongation falling within the above range is advantageous in improving the stretchability of the nonwoven fabric. Also when the nonwoven fabric is used as a bandage, the followability when the bandage is applied on an actively moving part such as a joint can be improved. The breaking elongation in the aforementioned at least one direction in the plane direction is normally less than or equal to 300%, preferably less than or equal to 250%. The breaking elongation is also measured by a tensile test in accordance with JIS L 1913 "Test methods for nonwoven s".

The aforementioned "at least one direction in the plane direction" can be the MD direction, and when the nonwoven fabric has a lengthwise direction and a widthwise direction as in the case of a bandage, "at least one direction in the plane direction" is preferably the lengthwise direction of the nonwoven fabric.

The breaking elongation in other directions than the aforementioned at least one direction in the plane direction, for example in the CD direction, or in the widthwise direction when the nonwoven fabric has the lengthwise direction and the widthwise direction as in the case of a bandage, is for example, 50 to 500%, preferably 100 to 350%.

The recovery rate after 50% extension for at least one direction in the plane direction (recovery rate after 50% extension) is preferably greater than or equal to 70% (less than or equal to 100%), more preferably greater than or equal to 80%, further preferably greater than or equal to 90%. When the recovery rate after 50% extension falls within this range, the followability to extension is improved, and for example, when the nonwoven fabric is used as a bandage, it becomes possible to sufficiently follow the form of the site where it is used, and to become advantageous for improving the self-adhesiveness by the friction between the overlapped nonwoven fabrics. In particular, when several nonwoven fabrics are overlapped as a result of winding, the fixing force by friction as a whole corresponds to the recovery stress, and a behavior similar to increasing the mass per unit area is exhibited. That is, if the extension recovery rate is small, the nonwoven fabric cannot follow motion when the site where it is used has a complicated shape or when it moves during use, and the part that has deformed by the motion of the body does not recover the original condition, and the fixation of the wound part is weakened.

The aforementioned "at least one direction in the plane direction" can be the MD direction, and when the nonwoven fabric has a lengthwise direction and a widthwise direction as in the case of a bandage, "at least one direction in the plane direction" is preferably the lengthwise direction of the nonwoven fabric.

Recovery rate after 50% extension is defined by the following formula:

$$\text{Recovery rate after 50\% extension (\%)} = 100 - X$$

(wherein X is a residual strain (%) after the test when the load is removed immediately after the extension percentage has reached 50% in a tensile test in accordance with JIS L 1913 "Test methods for nonwovens".)

The recovery rate after 50% extension in other directions than the at least one direction in the plane direction, for example, in the CD direction, or in a widthwise direction when the nonwoven fabric has a lengthwise direction and a widthwise direction as in the case of a bandage is for example, greater than or equal to 70% (less than or equal to 100%), preferably greater than or equal to 80%

The stress at 50% extension for at least one direction in the plane direction is preferably 3 to 50 N/50 mm, more preferably 4 to 40 N/50 mm, further preferably 5 to 30 N/50 mm. The stress at 50% extension falling within this range is advantageous for followability to elongation. The stress at 50% extension is also measured by a tensile test in accordance with JIS L 1913 "Test methods for nonwovens".

The aforementioned "at least one direction in the plane direction" can be the MD direction, and when the nonwoven fabric has a lengthwise direction and a widthwise direction as in the case of a bandage, "at least one direction in the plane direction" is preferably the lengthwise direction of the nonwoven fabric.

The stress at 50% extension in other directions than the at least one direction in the plane direction, for example, in the CD direction, or in a widthwise direction when the nonwoven fabric has a lengthwise direction and a widthwise direction as in the case of a bandage is for example, 0.5 to 50 N/50 mm, preferably 1 to 30 N/50 mm.

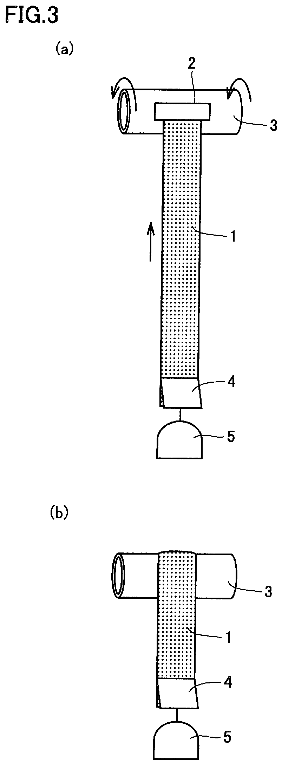

The nonwoven fabric according to the present invention is excellent in the self-adhesiveness, particularly in the self-adhesiveness of the broken end part. The self-adhesiveness enables latching or fixing of one nonwoven fabric part to other nonwoven fabric part by joining or intermingling at the time of contact between nonwoven fabrics without necessity of using an adhesive, a stopper or the like. The self-adhesiveness of the nonwoven fabric can be evaluated by curved surface sliding stress. The nonwoven fabric according to the present invention has a curved surface sliding stress in the part except the broken end part of, for example, greater than or equal to 0.5 N/50 mm, preferably greater than or equal to 1 N/50 mm, more preferably greater than or equal to 3 N/50 mm, further preferably greater than or equal to 5 N/50 mm, particularly preferably greater than the breaking strength. On the other hand, according to the present invention, it is possible to realize a curved surface sliding stress in the broken end part of greater than or equal to 5 N/50 mm, still further greater than or equal to 7 N/50 mm, yet still further greater than or equal to 10 N/50 mm. By selecting the above-described broken end part length D within a predetermined range, it is possible to improve the curved surface sliding stress (self-adhesiveness) in the broken end part. The curved surface sliding stress in other parts than the broken end part and the curved surface sliding stress in the broken end part are measured in accordance with the method described in the section of Examples using a tensile tester (Fig. 3 to Fig. 5).

The air permeability of the nonwoven fabric according to the present invention is greater than or equal to 0.1 $cm^3/cm^2\cdot sec$, for example, 1 to 500 $cm^3/cm^2\cdot sec$, preferably 5 to 300 $cm^3/cm^2\cdot sec$, more preferably about 10 to 200 $cm^3/cm^2\cdot sec$ by the air permeability according to the Frajour type method. When the air permeability falls within this range, the permeability is excellent and the nonwoven fabric is difficult to become sweaty, and is more suited for an application such as a bandage to be used for a human body.

(2) Structure and material of nonwoven fabric

As will be specifically described later, the nonwoven fabric according to the present invention contains crimped fibers that are crimped in a coiled form. Preferably, the nonwoven fabric according to the present invention has such a structure that crimped fibers forming the fabric do not substantially adhere to each other, but principally, the crimped fibers are intertwined at their crimped coil parts and thus they are restricted or latched. Also in the nonwoven fabric according to the present invention, preferably, most (majority) of the crimped fibers (the directions of axial core of crimped fibers) forming the fabric are oriented substantially parallel with the nonwoven fabric plane (sheet plane). In the description of the present application, the expression "oriented substantially parallel with the plane direction" means that the part where a large number of crimped fibers (the directions of axial core of crimped fibers) are locally oriented in the thickness direction does not exist repeatedly as in the case of the intermingling by the needle punching, for example.

The nonwoven fabric according to the present invention preferably includes crimped fibers that are oriented in the plane direction (longitudinal direction) thereof and crimped in a coiled form, and the neighboring or crossing crimped fibers are mutually intermingled at crimpled coil parts thereof. Also in the thickness direction (or diagonal direction) of the nonwoven fabric, preferably, crimped fibers are mutually intermingled lightly. Particularly, in a fiber web, fibers are intermingled in the course of shrinking into a coiled form, and crimped fibers are restricted by the intermingled crimped coil parts.

Therefore, the nonwoven fabric according to the present invention can be a stretchable nonwoven fabric capable of extending largely in the plane direction (longitudinal direction) by the intermingled crimped coil parts rather than in the widthwise direction and the thickness direction. In the stretchable nonwoven fabric, preferably, the crimped fibers are oriented in the plane direction and in the lengthwise direction, and thus when a tension is applied in the lengthwise direction, the intermingled crimped coil parts extend, and tend to recover the original coil form, so that the stretchable nonwoven fabric can exhibit high stretchability in the plane direction and the lengthwise direction. Further, by the light intermingling between crimped fibers in the thickness direction of the nonwoven fabric, the cushioning characteristics and the flexibility can emerge in the thickness direction, and thus the nonwoven fabric can have an excellent touch and feeling. Further, a crimped coil part easily intermingles with other crimped coil part by a contact at a certain degree of pressure. By this intermingling of the crimped coil parts, the self-adhesiveness can be emerged.

Preferably, the crimped fibers are oriented in the plane direction and the lengthwise direction, and hence the intermingled crimped coil parts extend by the elastic deformation when a tension is applied in the lengthwise direction, and finally come loose when a tension is further applied, so that excellent cutting characteristics (hand-cuttability) are realized. Thus, the nonwoven fabric according to the present invention can be provided with the self-adhesiveness, the hand-cuttability and the stretchability with a good balance.

In contrast to this, when the fibers that form the nonwoven fabric are not substantially fused to each other, and there are a lot of fibers oriented in the thickness directions (the direction perpendicular to the sheet plane), these fibers form coiled crimps, and the fibers are intertwined very complicatedly. As a result, other fibers are restricted or fixed more than needed, and stretching of the crimped coil parts forming the fibers are inhibited, and thus the stretchability of the nonwoven fabric is deteriorated. Therefore, it is desired to orient the crimped fibers parallel with the plane direction of the nonwoven fabric as much as possible.

Thus, the coiled crimped fibers are oriented substantially parallel with the plane direction of the nonwoven fabric, so that the nonwoven fabric according to the present invention can have stretchability in the plane direction. In contrast, when it is extended in the thickness direction, the fibers get loose relatively easily, so that the stretchability (contraction property) as seen in the plane direction does not emerge. Even when the fibers are dense, and the orientation is difficult to be observed visually, the orientation of the fibers can be checked easily by observing the stretchability in this manner.

As described above, the nonwoven fabric according to the present invention includes crimped fibers that are crimped in a coiled form. The crimped fibers are preferably oriented mainly in the plane direction of the nonwoven fabric, and preferably crimped substantially uniformly in the thickness direction. The crimped fibers are formed of a composite fiber in which a plurality of resins having different coefficients of thermal contraction (or coefficients of thermal expansion) form a phase structure.

The composite fiber forming the crimped fibers is a fiber (potential crimped fiber) having an asymmetry or layered (so-called bimetal) structure that will be crimped by heating due to the difference in coefficient of thermal contraction (or coefficient of thermal expansion) between a plurality of resins. The plurality of resins are different from each other normally in a softening point or a melting point. The plurality of resins can be selected from thermoplastic resins, for example, polyolefin resins (poly $C_{2-4}$ olefin resins such as low-density, medium-density, and high-density polyethylene, polypropylene); acryl resins (acrylonitrile resins having an acrylonitrile unit such as acrylonitrile-vinyl chloride copolymer); polyvinyl acetal resins (such as polyvinyl acetal resin); polyvinyl chloride resins (such as polyvinyl chloride, vinyl chloride-vinyl acetate copolymer, and vinyl chloride-acrylonitrile copolymer); polyvinylidene chloride resins (such as vinylidene chloride-vinyl chloride copolymer, and vinylidene chloride-vinyl acetate copolymer); styrene resins (such as heat resistant polystyrene); polyester resins (poly $C_{2-4}$ alkylene arylate resins such as polyethylene terephthalate resin, polytrimethylene terephthalate resin, polybutylene terephthalate resin, and polyethylene naphthalate resin); polyamide resins (aliphatic polyamide resins such as polyamide 6, polyamide 66, polyamide 11, polyamide 12, polyamide 610, and polyamide 612, semi-aromatic polyamide resins, and aromatic polyamide resins such as polyphenylene isophthalamide, polyhexamethylene terephthalamide, and poly p-phenylene terephthalamide); polycarbonate resins (such as bisphenol A polycarbonate); polyparaphenylene benzobisoxazole resin; polyphenylene sulfide resin; polyurethane resins; cellulose resins (such as cellulose ester) and so on. Further, each of these thermoplastic resins may contain other copolymerizable unit.

Among these, as the above plurality of resins, from the view point that the fibers do not melt or soften to be fused even when they are heat-treated with high-temperature water vapor, non-wet heat adhesive resins (or heat resistant hydrophobic resins or nonaqueous resins) having a softening point or a melting point of greater than or equal to 100°C, for example, polypropylene resins, polyester resins, and polyamide resins are preferred, and in particular, from the view point of the excellent balance of the heat resistance and the fiber formability, aromatic polyester resins, and polyamide resins are preferred. At least the resin that is exposed on the surface of the composite resin is preferably a non-wet heat adhesive resin so that the composite fiber (potential crimped fiber) forming the nonwoven fabric does not fuse when treated with high-temperature water vapor.

The plurality of resins forming the composite fiber are only required to have different coefficients of thermal contraction, and may be a combination of resins of the same system, or may be a combination of different resins.

From the view point of adhesion, the plurality of resins forming the composite fiber are preferably a combination of resins of the same system. In the case of a combination of resins of the same system, normally, a combination of component (A) forming a homopolymer (essential component), and component (B) forming a modified polymer (copolymer) is used. In other words, by modifying the homopolymer which is an essential component, for example, by copolymerizing a copolymerizable monomer that decreases the degree of crystallinity and the melting point or the softening point, the resultant copolymer can have a lower degree of crystallinity than the homopolymer or can be amorphous, or can have a lower melting point, softening point or the like than the homopolymer. By changing the crystallinity, the melting point or the softening point in this manner, it is possible to differentiate the coefficient of thermal contraction. The difference in the melting point or the softening point can be for example, 5 to 150°C, preferably 40 to 130°C, more preferably about 60 to 120°C. The percentage of the copolymerizable monomer used for modification with respect to the total monomer is, for example, 1 to 50 mol%, preferably 2 to 40 mol%, more preferably 3 to 30 mol% (particularly, 5 to 20 mol%). The mass ratio between the component forming the homopolymer and the component forming the modified polymer can be selected depending on the structure of the fibers, and is, for example, homopolymer component (A)/modified polymer component (B) = 90/10 to 10/90, preferably 70/30 to 30/70, more preferably 60/40 to 40/60.

For ease of manufacturing of the potentially crimping composite fiber, the composite fiber is preferably a combination of aromatic polyester resins, in particular, a combination of polyalkylene arylate resin (a), and a modified polyalkylene arylate resin (b). Polyalkylene arylate resin (a) can be a homopolymer of aromatic dicarboxylic acid (symmetric aromatic dicarboxylic acid such as terephthalic acid, and naphthalene-2,6-dicarboxylic acid) and an alkane diol component ($C_{2-6}$ alkane diol such as ethylene glycol and butylene glycol). Specifically, a poly $C_{2-4}$ alkylene terephthalate resin such as polyethylene terephthalate (PET) or polybutylene terephthalate (PBT) is used, and normally, PET used for a general PET fiber having an intrinsic viscosity of 0.6 to 0.7 is used.

On the other hand, in modified polyalkylene arylate resin (b), as a copolymerizing component that decreases the melting point or the softening point, and the crystallinity of polyalkylene arylate resin (a) which is an essential component, for example, a dicarboxylic acid component such as asymmetric aromatic dicarboxylic acid, alicyclic dicarboxylic acid, and aliphatic dicarboxylic acid, and an alkane diol component having a longer chain length than the alkane diol of polyalkylene arylate resin (a) and/or an ether bond-containing diol component can be recited. The copolymerizing component can be used solely or in combination of two or more kinds. Among these components, as a dicarboxylic acid component, asymmetric aromatic dicarboxylic acids (such as isophthalic acid, phthalic acid, and sodium 5-sulfoisophthalate), aliphatic dicarboxylic acids ($C_{6-12}$ aliphatic dicarboxylic acid such as adipic acid) and the like are commonly used, and as a diol component, alkane diols ($C_{3-6}$ alkane diols such as 1,3-propanediol, 1,4-butanediol, 1,6-hexanediol, and neopentyl glycol), polyoxyalkylene glycols (polyoxy $C_{2-4}$ alkylene glycols such as diethylene glycol, triethylene glycol, polyethylene glycol, and polytetramethylene glycol) and the like are commonly used. Among these, asymmetric aromatic dicarboxylic acids such as isophthalic acid, polyoxy $C_{2-4}$ alkylene glycols such as diethylene glycol and the like are preferred. Further, modified polyalkylene arylate resin (b) may be an elastomer made up of a $C_{2-4}$ alkylene arylate (such as ethylene terephthalate, and butylene terephthalate) as a hard segment, and a (poly)oxyalkylene glycol or the like as a soft segment.

In modified polyalkylene arylate resin (b), the percentage of the dicarboxylic acid component (for example, isophthalic acid) for decreasing the melting point or the softening point, relative to the total amount of the dicarboxylic acid component forming modified polyalkylene arylate resin (b) is for example, 1 to 50 mol%, preferably 5 to 50 mol%, more preferably 15 to 40 mol%. The percentage of the diol component (for example, diethylene glycol) for decreasing the melting point or the softening point, relative to the total amount of the diol component forming modified polyalkylene arylate resin (b) is, for example, less than or equal to 30 mol%, preferably less than or equal to 10 mol% (for example, 0.1 to 10 mol%). If the percentage of the copolymerizing component is too low, sufficient crimps do not emerge, and the shape stability and the stretchability of the nonwoven fabric after emergence of crimps are deteriorated. On the other hand, if the percentage of the copolymerizing component is too high, the crimp emerging performance is high, but it becomes difficult to conduct spinning stably.

Modified polyalkylene arylate resin (b) may contain a polyvalent carboxylic acid component such as trimellitic acid or pyromellitic acid, a polyol component such as glycerin, trimethylolpropane, trimethylolethane, or pentaerythritol and the like as a monomer component as is necessary.

The shape of the cross section of the composite fiber (the shape of the section perpendicular to the longitudinal direction of the fiber) may be a hollow section shape without limited to a round section or variant sections [such as flat, elliptical, polygonal, 3 to 14-foiled, T-shape, H-shaped, V-shaped, and dog-bone-like (I-shaped)] which are general solid section shapes, but it is normally a round section.

As a structure of the cross section of the composite fiber, a phase structure formed by a plurality of resins, for example, structures of a core-clad type, a sea-island type, a blend type, a parallel type (side-by-side type or multilayer bonding type), a radial type (radial bonding type), a hollow radial type, a block type, a random composite type and the like can be recited. Among these, the structure in which the phase parts neighbor (a so-called bimetal structure) and the structure in which the phase structure is asymmetric, for example, an eccentric core-clad type, or parallel type structure is preferred for ease of causing emergence of self-crimping by heating.

When the composite fiber has a core-clad type structure such as an eccentric core-clad type, the core part may be formed of a wet heat adhesive resin (for example, vinyl alcohol polymers such as ethylene-vinyl alcohol copolymer and polyvinyl alcohol), or a thermoplastic resin having a low melting point or softening point (for example, polystyrene, low-density polyethylene or the like) insofar as it has a difference in thermal contraction with the non-wet heat adhesive resin of the clad part situated on the surface, and can crimp.

The mean fineness of the composite fiber is selected from the range of 0.1 to 50 dtex, and is preferably 0.5 to 10 dtex, more preferably 1 to 5 dtex (particularly, 1.5 to 3 dtex). If the fineness is too small, it becomes difficult to manufacture the fiber itself, and additionally it is difficult to ensure the strength of the fiber. Also in the step of causing emergence of crimps, it becomes difficult to allow emergence of neat coiled crimps. On the other hand, if the fineness is too large, the fiber becomes rigid, and sufficient crimps are difficult to emerge.

The mean fiber length of the composite fiber is selected from the range of 10 to 100 mm, and is preferably 20 to 80 mm, more preferably 25 to 75 mm (particularly 40 to 60 mm). If the fiber length is too short, it becomes difficult to form a fiber web, and also intermingling between crimped fibers is insufficient when crimps are caused to emerge, so that it becomes difficult to ensure the strength and the stretchability of the nonwoven fabric. If the fiber length is too long, it becomes difficult to form a fiber web of a uniform mass per unit area, and also intermingling between fibers frequently emerges at the point of time of web formation, and they interfere with each other in emergence of crimps to make emergence of stretchability difficult. When the mean fiber length falls within the above range, part of the fibers crimped on the surface of the nonwoven fabric is exposed appropriately on the surface of the nonwoven fabric, so that it is possible to improve the self-adhesiveness of the nonwoven fabric. Further, the mean fiber length within the above range is advantageous in obtaining excellent hand-cuttability.

The composite fiber is a potential crimped fiber, and by subjecting the composite fiber to a heat treatment, crimps emerge (or appear), and the composite fiber becomes a fiber having substantially coiled (spiral or helical spring) spatial crimps. The number of crimps before heating (machine crimp number) is 1 to 25 crimps/25 mm, more preferably 5 to 20 crimps/25 mm. The number of crimps after heating is, for example, greater than or equal to 30 crimps/25 mm (for example, 30 to 200 crimps/25 mm), preferably 35 to 150 crimps/25 mm, more preferably about 40 to 120 crimps/25 mm, and may be 45 to 120 crimps/25 mm (in particular, 50 to 100 crimps/25 mm).

In the nonwoven fabric according to the present invention, it is preferred that the crimped fibers are crimped substantially uniformly in the thickness direction, in other words, crimps of the composite fiber emerge substantially uniformly in the thickness direction. Specifically, in a center part (internal layer) of the regions obtained by dividing into three equal parts in the thickness direction in the cross section in the thickness direction, the number of fibers forming a coiled crimp of one round or more is preferably 5 to 50 fibers/5 mm (length in the plane direction) · 0.2 mm (thickness), more preferably 10 to 50 fibers/5 mm (plane direction) · 0.2 mm (thickness), further preferably 20 to 50 fibers/5 mm (plane direction) · 0.2 mm (thickness). Since axes of most of the crimped fibers are oriented substantially parallel with the plane direction, and the number of crimps is substantially uniform in the thickness direction, high stretchability is realized even though a rubber or an elastomer is not contained, and practical strength can be imparted even though an adhesive is not contained. By the wording "regions obtained by dividing into three equal parts in the thickness direction" used in the description of the present application, each region obtained by slicing into three equal parts in the direction orthogonal to the thickness direction of the nonwoven fabric is referred.

The uniformity of the crimps in the thickness direction can also be evaluated by the uniformity of the curvature of fiber. The curvature of fiber means a ratio (L2/L1) of a fiber length (L2) to distance (L1) between both ends of a crimped fiber, and the curvature of fiber (in particular, the curvature of fiber in the middle region in the thickness direction) is, for example, greater than or equal to 1.3 (for example, 1.35 to 20), preferably 2 to 10 (for example, 2.1 to 9.5), more preferably 4 to 8 (in particular, 4.5 to 7.5). As will be described later, since the curvature of fiber is measured based on an electron microphotograph of the section of the nonwoven fabric, fiber length (L2) does not mean a fiber length (actual length) of the three-dimensionally crimped fiber in an extended and straightened condition, but means a fiber length (fiber length in the photograph) of the two-dimensionally crimped fiber in an extended and straightened condition on the photograph. Therefore, the fiber length (L2) is measured to be smaller than the actual fiber length.

When crimps emerge substantially uniformly in the thickness direction, the curvature of fiber is uniform in the thickness direction. The uniformity of the curvature of fiber can be evaluated by comparison of curvature of fiber among individual layers obtained by dividing into three equal parts in the thickness direction in the section of the thickness direction. In other words, in the section of the thickness direction, the curvature of fiber in each region obtained by dividing into three equal parts in the thickness direction falls within the above range, and a percentage of the minimum value to the maximum value of the curvature of fiber in each region (percentage of the region where the curvature of fiber is minimum to the region where the curvature of fiber is maximum) is, for example, greater than or equal to 75% (for example, 75 to 100%), preferably 80 to 99%, more preferably about 82 to 98% (in particular, 85 to 97%).

As a specific measuring method for curvature of fiber and uniformity thereof, a method of imaging the section of the nonwoven fabric by an electron microphotograph, and measuring curvature of fiber for a region selected from the individual regions obtained by dividing into three equal parts in the thickness direction is employed. The region to be measured is a region of greater than or equal to 2 mm in the lengthwise direction for each layer of a front layer (front region), an internal layer (middle region), and a back layer (back region) obtained by dividing into three equal parts. The thickness direction of each measurement region is set in such a manner that each measurement region has the same extension of thickness near the center of each layer. Also each measurement region is set to contain greater than or equal to 100 (preferably greater than or equal to 300, more preferably about 500 to 1000) fiber fragments that are parallel in the thickness direction and for which curvature of fiber can be measured in each measurement region. After setting each of these measurement regions, the curvature of fiber of every fiber in the region is measured, and a mean value is calculated for each measurement region, and then uniformity of the curvature of fiber is calculated by comparing the region showing the maximum mean value and the region showing the minimum mean value.

The crimped fibers forming the nonwoven fabric have substantially coiled crimps after emergence of crimps as described above. The mean radius of curvature of the circle formed by the coil of the crimped fiber can be selected, for example, from the range of about 10 to 250 μm, and is preferably 20 to 200 μm (for example, 50 to 200 μm), more preferably 50 to 160 μm (for example, 60 to 150 μm), further preferably 70 to 130 μm. The mean radius of curvature is an index indicating the mean size of the circles formed by the coil of the crimped fiber, and the large value of the mean radius of curvature means that the formed coil has a loose shape, or in other words, it has a shape having a small number of crimps. A small number of crimps is disadvantageous for emergence of sufficient stretching performance because intermingling between crimped fibers is reduced, and the shape recovery for the deformed coil shape becomes difficult. When the mean radius of curvature is too small, intermingling between crimped fibers is not insufficient, and it becomes difficult to ensure the web strength. In such a case, the stress at the time of deformation of the shape of the coil is too large, and the breaking strength is excessively large, so that it becomes difficult to obtain an appropriate stretchability.

The mean pitch (mean crimping pitch) of the coil in the crimped fibers is, for example, 0.03 to 0.5 mm, preferably 0.03 to 0.3 mm, more preferably 0.05 to 0.2 mm. If the mean pitch is excessively large, the number of coiled crimps that can emerge per one fiber fragment is small, and sufficient stretchability cannot be exerted. If the mean pitch is excessively small, intermingling between crimped fibers is not sufficient, and it becomes difficult to ensure the strength of the nonwoven fabric.

The nonwoven fabric (fiber web) may contain other fiber (non-composite fiber) in addition to the above composite fiber. As the non-composite fiber, for example, besides the aforementioned fibers formed of a non-wet heat adhesive resin or a wet heat adhesive resin, cellulose fibers [for example, natural fibers (cotton, wool, silk, linen, etc.), semisynthetic fibers (acetate fibers such as triacetate fiber), regenerated fibers (rayon, polynosic, cupra, Lyocell (for example, registered tradename: "Tencel", etc.))] can be recited. Mean fineness and mean fiber length of the non-composite fiber are similar to those of the composite fiber. The non-composite fiber can be used solely or in combination of two or more kinds. Among these, regenerated fibers such as rayon, semisynthetic fibers such as acetate, polyolefin fibers such as polypropylene fiber or polyethylene fiber, polyester fibers, polyamide fibers and the like are preferred. In particular, from the view point of the blendability or the like, the fiber of the same type as the composite fiber is preferred, and, for example, when the composite fiber is a polyester fiber, the non-composite fiber can also be a polyester fiber.

The ratio between the composite fiber and the non-composite fiber (mass ratio) can be selected from the range of composite fiber/non-composite fiber = 50/50 to 100/0, and is, for example, 60/40 to 100/0 (for example, 60/40 to 99.5/0.5), preferably 70/30 to 100/0 (for example, 70/30 to 99.5/0.5), more preferably 80/20 to 100/0 (for example, 80/20 to 99.5/0.5), further preferably 90/10 to 100/0 (for example, 90/10 to 99.5/0.5), particularly preferably 95/5 to 100/0. By cotton blending of the non-composite fiber, it is possible to adjust the balance of the strength and the stretchability or the flexibility of the nonwoven fabric. However, if the proportion of the composite fiber is too small, in stretching of the composite fiber after emergence of crimps, in particular, in contraction of the composite fiber after extension, the non-composite fiber resists the contraction, so that recovery of the shape of the nonwoven fabric becomes difficult.

The nonwoven fabric (fiber web) may contain commonly used additives, for example, stabilizers (a heat stabilizer such as a copper compound, an ultraviolet absorber, a light stabilizer, an antioxidant, etc.), antibacterial agents, deodorizing agents, perfumes, coloring agents (dyes and pigments etc.), fillers, antistatic agents, flame retarders, plasticizers, lubricants, crystallization speed retarders and so on. The additive can be used solely or in combination of two or more kinds. The additive may be carried on the surface of the fibers, or can be contained in the fibers.

<Method for producing nonwoven fabric>

The nonwoven fabric according to the present invention is produced by a method including the step of webbing fibers including the composite fiber (potential crimped fiber) (webbing step), and the step of heating the fiber web to crimp the composite fiber (heating step).

As a method for forming a fiber web in the webbing step, commonly used methods, for example, direct methods such as a spun bond method, and a melt blow method, a CADe methods using melt-blown fibers, staple fibers or the like, and a dry method such as an airlaying method can be employed. Among these, a carding method using melt-blown fibers or staple fibers, in particular, a carding method using staple fibers is generally used. Examples of webs obtained by using staple fibers include a random web, a semi-random web, a parallel web, and a cross lap web.

Prior to the heating step, an entangling step for entangling at least part of the fibers in the fiber web is conducted. By conducting the entangling step, it is possible to obtain a nonwoven fabric in which crimped fibers are appropriately intermingled in the next heating step. The entangling method is a method of entangling by spraying or injecting (blowing) water is preferred. Entangling the fibers by water flow is advantageous in increasing the density of intermingling by crimping in the heating step. The water to be sprayed or injected may be sprayed from one side of the fiber web, or may be sprayed from both sides, however, from the view point of effectively conducting strong intermingling, the water is preferably sprayed from both sides.

The jet pressure of the water in the entangling step is, for example, greater than or equal to 2 MPa (for example, 2 to 15 MPa), preferably 3 to 12 MPa, more preferably 4 to 10 MPa (in particular, 5 to 8 MPa) so that the fiber intermingling falls within an appropriate range. The temperature of the water to be sprayed or injected is, for example, 5 to 50°C, preferably 10 to 40°C, for example, 15 to 35°C (normal temperature).

As a method for spraying or injecting water, a method of injecting water by using a nozzle or the like having a regular spraying area or spraying pattern is preferred from the view point of the convenience or the like. Specifically, water can be injected to the fiber web that is transferred by a belt conveyer in the condition that the fiber web is placed on the conveyer belt. The conveyer belt may be water-permeable, and also water may be injected to the fiber web through the water-permeable conveyer belt from the back side of the fiber web. In order to control scattering of fibers by injection of water, the fiber web may be moistened in advance with a small amount of water.

In the nozzle for spraying or injecting water, a plate or a die in which predetermined orifices are successively arranged in the widthwise direction can be used and arranged so that the orifices are aligned in the widthwise direction of the fed fiber web. As the orifice line, at least one line is required, and a plurality of lines may be arranged in parallel. Also, a plurality of nozzle dies each having one line of orifices may be installed in parallel.

In the case of using a nozzle of the type in which a plate is punched to give orifices, the thickness of the plate can be, for example, 0.5 to 1.0 mm. The diameter of an orifice is normally 0.01 to 2 mm, preferably 0.05 to 1.5 mm, more preferably 0.1 to 1.0 mm. The pitch of orifices is normally 0.1 to 2 mm, preferably 0.2 to 1.5 mm, more preferably 0.3 to 1 mm.

Basically, the belt conveyer used herein is not particularly limited insofar as it can convey a fiber web without disturbing the form of the fiber web; however, an endless conveyer is desirably used. Only one belt conveyer may be used alone, or another belt conveyer may be combined as is necessary, and a fiber web may be conveyed while it is sandwiched between these belts. In particular, in the next heating step for fixing the fiber web in the final form, the fiber web may be sandwiched between a set of belts, and the density of the fiber web may be adjusted. By conveying in this manner, it is possible to prevent the form of the conveyed web from deforming by the water for entangling, the high-temperature water vapor in the heating step, and external force such as oscillation of the conveyer in treating the fiber web. When one set of belts is used, the distance between the belts can be appropriately selected according to the mass per unit area and the density of the desired fiber web, and is for example, 1 to 10 mm, preferably 1 to 8 mm, more preferably 1 to 5 mm.

The endless belt used in the conveyer is not particularly limited insofar as it does not interfere the conveyance of the fiber web, water for entangling, and a high-temperature water vapor treatment in the heating step, and when it is a net, a net that is coarser than approximately 90 mesh (for example, net of about 10 to 80 mesh) is preferred. A net with finer mesh has poor air permeability, so that the water for entangling and the water vapor in the next step become difficult to permeate. While the material of the belt is not particularly limited, as the material of the belt used in the heating step, metal, thermo-protected polyester resins, heat resistant resins such as polyphenylene sulfide resins, polyarylate resins (wholly aromatic polyester resins), and aromatic polyamide resins and the like are preferred from the view point of heat resistance against the water vapor treatment. While the belt used in the conveyer may be the same in the entangling step by water flow or the like, and in the heating step by the high-temperature water vapor, normally separated different conveyers are used because adjustment is required in each step.

Prior to the above entangling step, a step of making fibers in the fiber web be localized in the plane (localizing step) is provided. By conducting this step, a region where the fiber density is low is formed in the fiber web, so that it is possible to efficiently inject the water flow inside the fiber web in the case where the entangling step is water flow entangling, and it becomes easy to realize appropriate intermingling not only on the surface of the fiber web but also inside the fiber web.

The localizing step is conducted by spraying or injecting low pressure water to the fiber web. The low pressure water may be sprayed or injected to the fiber web continuously, but is sprayed intermittently or periodically. By spraying water intermittently or periodically to the fiber web, it is possible to form a plurality of low-density parts and a plurality of high-density parts alternately and periodically.

The ejection pressure of the water in this localizing step is 0.1 to 1.5 MPa, preferably 0.3 to 1.2 MPa, more preferably about 0.6 to 1.0 MPa. The temperature of the water to be sprayed or injected is, for example, 5 to 50°C, preferably 10 to 40°C, for example, 15 to 35°C (normal temperature).

The method for spraying or injecting water intermittently or periodically is not particularly limited insofar as the method enables periodic and alternate formation of the gradient of the density in the fiber web, however, from the view point of convenience or the like, a method of spraying water through a plate-like member (porous plate or the like) having a regular spraying or a spraying pattern formed by a plurality of pores is preferred.

Specifically, the fiber web obtained in the webbing step is fed to the next step by the belt conveyer, and then the fiber web may be allowed to pass between a drum formed of a porous plate (porous plate drum) and the belt in the condition that it is placed on the conveyor belt. The conveyor belt may be water-permeable, and when the fiber web passes between the porous plate drum and the belt, water can be ejected in a spray form so that the water passes the conveyer belt through the fiber web from inside the drum. In this manner, the fibers forming the fiber web on the conveyor belt can be moved to the non-spraying area where no pore of the porous plate is allocated, so that it is possible to reduce the fiber quantity of the site to which a porous is allocated.

While the arrangement or the arrangement structure of the pores of the porous plate is not particularly limited, it may have, for example, a structure in which pores are arranged alternately in a net or grid (hound's tooth check) pattern. The pore diameter of each pore is normally identical, and is, for example, 1 to 10 mm, preferably 1.5 to 5 mm. The pitch between neighboring pores is also normally identical, and is, for example, 1 to 5 mm, preferably 1.5 to 3 mm.

If the pore diameter is too small, the amount of flowing water is reduced, and there arises a case that the fibers of the fiber web cannot be moved. On the other hand, if the pore diameter is too large, the necessity of increasing the pitch arises for ensuring the shape of the drum, and as a result, there arises a part where water does not come into contact with the fiber web. This can raise quality unevenness or difficulty of conducting a uniform treatment. If the pitch of pores is too small, the necessity of decreasing the pore diameter inevitably arises, and the water amount cannot be ensured. Contrarily, if the pitch is too large, a part where water does not come into contact with the fiber web arises, and quality unevenness is likely to occur.

In the heating step, the fiber web is fed to the next step by the belt conveyer, and crimped by heating with high-temperature water vapor. In the method of treating with high-temperature water vapor, the fiber web fed by the belt conveyer is exposed to a high-temperature or superheated steam (high pressure steam) flow, and thus coil crimping occurs in the composite fiber (potential crimped fiber). In other words, by emergence of crimps, the composite fiber moves while its form is changed to a coil form, and three-dimensional intermingling among fibers emerges. Since the fiber web has air permeability, the high-temperature water vapor penetrates inside even if the treatment is conducted from one direction, and crimps that are substantially uniform in the thickness direction emerge, and fibers are intermingled uniformly.

Specifically, the fiber web is subjected to a treatment with high-temperature water vapor on the belt conveyer, and the fiber web contracts simultaneously with the high-temperature water vapor treatment. Therefore, it is desired that the fiber web to be fed is overfed in accordance with the areal contraction coefficient of the intended nonwoven fabric directly before exposure to the high-temperature water vapor. The rate of the overfeeding is 110 to 300%, preferably 120 to 250% relative to the length of the intended nonwoven fabric.

For supplying the fiber web with water vapor, a commonly used water-vapor injecting device is used. As the water-vapor injecting device, a device capable of spraying water vapor at a desired pressure and in a desired amount over the entire width of the fiber web almost uniformly is preferred. When a combination of two belt conveyers is used, the water-vapor injecting device is attached in one conveyer, and the water vapor is supplied to the fiber web through the water-permeable conveyor belt, or through the conveyor net placed on the conveyer.

A suction box may be attached to the other conveyer. While the excessive water vapor having passed the fiber web may be sucked and discharged by the suction box, it is preferred to supply the water vapor without being sucked and discharged by the suction box because the fiber web is required to be kept in a free state as much as possible so as to bring the water vapor into contact with the fiber web sufficiently and to make fiber crimps emerge by this heat emerge more efficiently. For conducting the water-vapor treatment on both the front and the back sides of the fiber web at once, another water-vapor injecting device may be installed in the conveyer of the downstream side than the site where the water-vapor injecting device is attached in the conveyer opposite to the conveyer to which the water-vapor injecting device is attached. When one wants to treat both the front and the back sides of the nonwoven fabric with water vapor in the case where the water-vapor injecting device on the downstream side is absent, the fiber web having once treated may be passed again in the treating device after the fiber web is turned over, as an alternative.

Since the high-temperature water vapor injected from the water-vapor injecting device is an airflow, it enters inside the fiber web without significantly moving the fibers in the fiber web which is an object to be treated unlike the case of the water flow entangling treatment or the needle punching treatment. It is considered that by the entry action of the water vapor flow into the fiber web, the water vapor flow efficiently covers the surface of each fiber existing in the fiber web, and enables uniform thermal crimping. Also since heat can be conducted inside the fiber web sufficiently, as compared with a dry heat treatment, the degree of crimping is almost uniform in the plane direction and the thickness direction.

Also as a nozzle for injecting the high-temperature water vapor, likewise the nozzle for water flow entangling, a plate or a die in which predetermined orifices are successively arranged in the widthwise direction can be used and arranged so that the orifices are aligned in the widthwise direction of the fed fiber web. As the orifice line, at least one line is required, and a plurality of lines may be arranged in parallel. Also, a plurality of nozzle dies each having one orifice line may be installed in parallel.

In the case of using a nozzle of the type in which a plate is punched to give orifices, the thickness of the plate may be about 0.5 to 1.0 mm. While the diameter and the pitch of orifices are not particularly limited insofar as emergence of intended crimps and fiber intermingling in association with this emergence can be efficiently achieved, the diameter of an orifice is normally 0.05 to 2 mm, preferably 0.1 to 1 mm, more preferably about 0.2 to 0.5 mm. The pitch of orifices is normally 0.5 to 5 mm, preferably 1 to 4 mm, more preferably about 1 to 3 mm. If the diameter of the orifice is too small, the operational problem of easily clogging is likely to occur. Contrarily, if it is too large, it becomes difficult to obtain a sufficient water vapor injecting force. On the other hand, if the pitch is too small, the pore diameter is also small, and the amount of high-temperature water vapor decreases. On the other hand, if the pitch is too large, it becomes difficult to ensure the strength because there arises a case that the high-temperature water vapor fails to hit the fiber web sufficiently.

Also the high-temperature water vapor to be used is not particularly limited insofar as emergence of intended fiber crimps and appropriate fiber intermingling in association with this can be achieved, and can be set according to the quality of material and form of the fiber to be used, and the pressure is, for example, 0.1 to 2 MPa, preferably 0.2 to 1.5 MPa, more preferably 0.3 to 1 MPa. If the pressure of the water vapor is too high, the fibers forming the fiber web can move more than required to cause disturbance of the formation, or the fibers can be intermingled more than required. In the extreme case, the fibers are fused together, and it becomes difficult to ensure the stretchability. Further, when the pressure is too weak, it becomes impossible to give the quantity of heat that is required for emergence of crimps of fibers to the fiber web, or the water vapor cannot penetrate the fiber web and emergence of crimps of fibers in the thickness direction tends to be nonuniform. Also it is difficult to control the uniform ejection of the water vapor from the nozzle.

The temperature of the high-temperature water vapor is, for example, 70 to 150°C, preferably 80 to 120°C, more preferably 90 to 110°C. The treatment speed with the high-temperature water vapor is, for example, less than or equal to 200 m/min., preferably 0.1 to 100 m/min., more preferably 1 to 50 m/min.

After causing emergence of crimps of the composite fiber in the fiber web in the manner as described above, there is sometimes a case that water remains in the nonwoven fabric, and hence, the nonwoven fabric may be dried as is necessary. Regarding the drying, it required that the fibers on the surface of the nonwoven fabric being in contact with the heater for drying will not be fused by the heat for drying and deteriorate the stretchability, and a commonly used method can be employed insofar as the stretchability can be maintained. While large-sized drying equipment such as a cylinder dryer, a tenter or the like used for drying nonwoven fabrics may be used, it is preferred to use non-contact methods such as far infrared radiation, microwave radiation, and electron beam radiation, a method of blowing hot air, a method of passing in hot air and the like because the remaining water is very small in amount, and is often in such a level that can be dried by relatively light drying means.

The obtained nonwoven fabric is wetted with water in its manufacturing process, and exposed under a high-temperature water vapor atmosphere. In other words, in the nonwoven fabric of the present invention, since the nonwoven fabric itself experiences a treatment similar to laundry, the extraneous matters adhered to the fibers such as fiber spinning oil are washed out. Therefore, the stretchable nonwoven fabric of the present invention is hygienic and exhibits high water repellency.

EXAMPLES

Hereinafter, the present invention will be described more specifically by way of examples, however, it is to be noted that the present invention is not limited by these examples. Physical property values in nonwoven fabric (bandage) obtained in the following Examples and Comparative Examples were determined by the following methods. The measuring results are shown in Table 1 to Table 5.

[1] Machine crimp number

[0104] The machine crimp number (number/25 mm) was measured in accordance with JIS L 1015 "Test methods for man-made staple fibers" (8.12.1).

[2] Mean number of coiled crimps

From a nonwoven fabric, a crimped fiber (composite fiber) was pulled out with the care not to extend the coiled crimp, and the mean number of coiled crimps (number/mm) was measured in accordance with JIS L 1015 "Test methods for man-made staple fibers" (8.12.1) as with the measurement of the machine crimp number. This measurement was conducted only for a fiber in which coiled crimps emerge.

[3] Mean crimping pitch

At the time of measuring the mean number of coiled crimps, the distance between successively neighboring coils was measured, and the mean crimping pitch ($\mu$m) was determined as a mean value of n = 100.

[4] Mean radius of curvature

Using a scanning electron microscope (SEM), a photograph of an arbitrary section of the nonwoven fabric, enlarged 100 times was taken. Among the fibers in the photograph of the nonwoven fabric section thus taken, for a fiber that forms a spiral (coil) of one or more rounds, the radius of a circle when the circle is described along the spiral (radius of the circle when the crimped fiber is observed in the coil axial direction) was determined as radius of curvature ($\mu$m). When a fiber describes a spiral ovally, 1/2 of the sum of the major axis and the minor axis of the oval was determined as radius of curvature. However, for excluding the case where sufficient coiled crimps do not emerge in the crimped fiber, or the case where the spiral form of the fiber is seen as an oval because it is observed diagonally, only the ovals having a ratio between the major axis and the minor axis of the oval falls within the range of 0.8 to 1.2 were selected as objects to be measured. The mean radius of curvature ($\mu$m) was determined as a mean value of n = 100.

[5] Curvature of fiber and uniformity of crimped fibers (composite fiber)

An electron microphotograph (magnification: 100-power) in an arbitrary section of a nonwoven fabric was taken, and in the part where the photographed fibers can be seen, the part was divided into three equal regions: a front layer, an internal layer, and a back layer in the thickness direction, and a measurement region was set in the vicinity of the center of each layer in such a manner that 500 or more crimped fibers that are greater than or equal to 2 mm in the lengthwise direction and measurable are contained. For these regions, an end-to-end distance (shortest distance) between one end and the other end of the crimped fiber was measured, and further, the fiber length (fiber length on the photograph) of the crimped fiber was measured. That is, when an end of a crimped fiber is exposed on the surface of the nonwoven fabric, the end is directly regarded as an end for measuring an end-to-end distance, and when an end is buried inside the nonwoven fabric, the boundary part at which the fiber is buried inside the nonwoven fabric (end on the photograph) is regarded as an end for measuring an end-to-end distance. Among the crimped fibers photographed at this time, the fiber image for which continuity of greater than or equal to 100 $\mu$m could not be recognized was excluded from objects to be measured. As a ratio (L2/L1) of fiber length (L2) of the composite fiber to the end-to-end distance (L1), curvature of fiber was calculated. A mean value of curvature of fiber was calculated for each of the front layer, the internal layer, and the back layer obtained by dividing into three equal parts in the thickness direction, and further from the ratio of the minimum value to the maximum value (minimum value/maximum value) among these, uniformity of the curvature of fiber in the thickness direction was calculated.

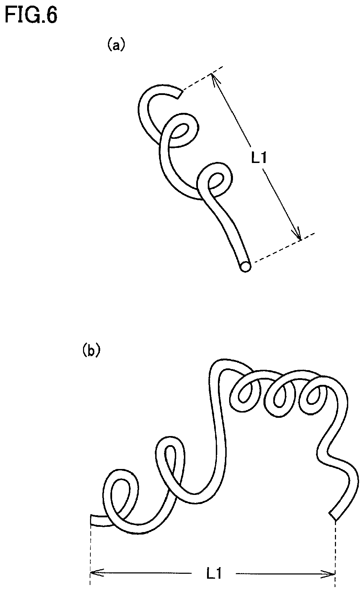

Fig. 6 shows a schematic view about the method for measuring curvature of fiber of a photographed crimped fiber. Fig. 6(a) shows a crimped fiber in which one end is exposed to the surface, and the other end is buried inside the nonwoven fabric, and in this case, end-to-end distance L1 is a distance from the end of the crimped fiber to the boundary part at which the fiber is buried inside the nonwoven fabric. On the other hand, fiber length L2 is a length of the fiber of the observable part of the crimped fiber (the part from the end of the crimped fiber to the point where it is buried inside the nonwoven fabric) extended two-dimensionally on the photograph.

Fig. 6(b) shows a composite fiber in which both ends are buried inside the nonwoven fabric, and in this case, end-to-end distance L1 is a distance between both ends in the part exposed on the surface of the nonwoven fabric (both ends on the photograph). On the other hand, fiber length L2 is a length of the crimped fiber of the part exposed on the surface of the nonwoven fabric extended two-dimensionally on the photograph.

[6] Mass per unit area

The mass per unit area of a nonwoven fabric as a whole, namely, the mean mass per unit area (g/m$^2$) of a nonwoven fabric was measured in accordance with JIS L 1913 "Test methods for nonwovens".

[7] Thickness and density

The thickness (mm) of a nonwoven fabric was measured in accordance with JIS L 1913 "Test methods for nonwovens", and the density (g/cm$^2$) of a nonwoven fabric was calculated from this value and the mass per unit area measured in the method of [6].

[8] Breaking strength, breaking elongation and stress at 50% extension

Measurement was conducted in accordance with JIS L 1913 "Test methods for nonwovens". The breaking strength (N/50 mm), the breaking elongation (%) and the stress at 50% extension (N/50 mm) were measured for the machine (MD) direction and the width (CD) direction of the nonwoven fabric.

[9] Recovery rate after 50% extension

A tensile test in conformity with JIS L 1913 "Test methods for nonwovens" was conducted, and the recovery rate after 50% extension was determined based on the following formula:

$$\text{Recovery rate after } 50\% \text{ extension (\%)} = 100 - X$$

In the formula, X represents the residual strain (%) after the test when a load is removed immediately after the extension has reached 50% in the tensile test. Recovery rate after 50% extension was measured for the MD direction and the CD direction.

[10] Compressive stress

Five sheets of nonwoven fabric were stacked, and the stress when they were compressed 20% in the thickness direction was measured, as the compressive stress (kPa). For measurement, a precision universal tester ("Autograph AG-IS" available from SHIMADZU CORPORATION) was used. For compression, a cylindrical compressing element having a diameter of 29 mm was used, and compression was conducted at a head speed of 10 mm/min.

[11] Broken end part length D

For the breakage sample obtained by the measurement of breaking strength in the above [8], broken end part length D (mm) was determined according to the aforementioned measuring method (see Fig. 1). The breaking direction is the MD direction, and is a lengthwise direction of the obtained nonwoven fabric having a lengthwise direction and a widthwise direction.

[12] Mass per unit area ratio of divisional areas

For the obtained nonwoven fabric having a lengthwise direction and a widthwise direction, a rectangular area having a length in the lengthwise direction of 5 cm and a length in the widthwise direction equivalent to the overall width of the nonwoven fabric was selected, and for this rectangular area, the ratio $W_i/W_{i+1}$ between the mass per unit area $W_i$ of the i-th divisional area and the mass per unit area $W_{i+1}$ of the (i+1)th divisional area was determined according to the aforementioned description (see Fig. 2). The mass per unit area ratio $W_i/W_{i+1}$ was determined by cutting out each divisional area (length in the lengthwise direction: 5 cm, length in the widthwise direction: 1 cm) contained in the rectangular area with scissors, and measuring the weight thereof, and calculating the ratio between these.
For determination of the mass per unit area ratio of divisional area, three kinds of nonwoven fabrics having an overall width of 5 cm, 10 cm, and 20 cm, respectively were prepared, and for each nonwoven fabric, the mass per unit area ratio $W_i/W_{i+1}$ of each divisional area contained in the rectangular area was determined. Further, for each of the three kinds of nonwoven fabrics, rectangular areas were selected at three points. The rectangular areas of these three points were selected so that the distance in the lengthwise direction (MD direction) of the nonwoven fabric between the neighboring rectangular areas was 1 m.
In Table 2, three rectangular areas respectively selected from the three kinds of nonwoven fabrics having different overall widths are referred to as rectangular areas 1, 2, and 3, respectively. In Table 2, the maximum value and the minimum value of the mass per unit area ratio $W_i/W_{i+1}$ in each of rectangular areas 1 to 3 are described, and the case where the mass per unit area ratio $W_i/W_{i+1}$ is 0.9 to 1.1 for every i is evaluated as "Evaluation A", and the case where the mass per unit area ratio $W_i/W_{i+1}$ is not 0.9 to 1.1 for any one or more i is evaluated as "Evaluation B". The nonwoven fabric having an overall width of, for example, 5 cm has five divisional areas in each of the rectangular areas 1 to 3, and the total number of i is 4. In each rectangular area, the total number of i is the total number of divisional areas -1. Weights of individual divisional areas measured for calculating the mass per unit area ratio $W_i/W_{i+1}$ are shown in Tables 3 to 5.

[13] Curved surface sliding stress

A nonwoven fabric was cut into a size of 50 mm wide × 600 mm long so that the MD direction was lengthwise direction, to prepare a sample 1. Then, as shown in Fig. 3(a), one end part of sample 1 was fixed to a winding core 3 (pipe roll made of polypropylene resin having an outer diameter of 30 mm and a length of 150 mm) with a one-side adhesive tape 2, and on the other end part of sample 1, a weight 5 of 150 g was attached so that uniform load was applied over the entire width of sample 1 by using an alligator clip 4 (clipping width 50 mm, a rubber sheet of 0.5 mm thick was fixed inside the opening with a double-stick tape before use).

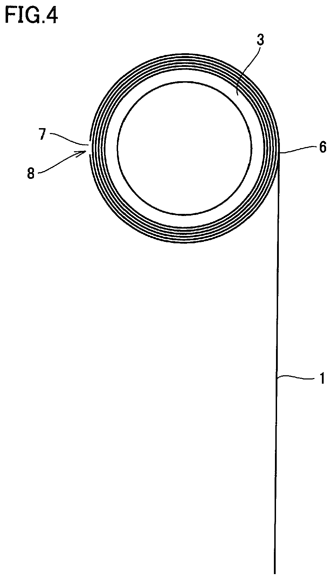

Then in the condition that winding core 3 on which sample 1 was fixed was lifted up so that sample 1 and weight 5 were hanging, winding core 3 was rotated five laps in such a manner that weight 5 did not largely swing to wind up sample 1, and weight 5 was lifted up (see Fig. 3(b)). In this condition, from a base point 6 which is a contact point (boundary between the part of sample 1 wound around winding core 3, and the part of sample 1 which is in a vertical state due to the gravity of weight 5) between a cylindrical part in the outermost peripheral part of sample 1 wound around winding core 3, and a plane part of sample 1 that is not wound around winding core 3, alligator clip 4 and weight 5 were slowly removed so that base point 6 did not move and deviate. Then at a point 7 positioned at a half lap (180°) along sample 1 wound around winding core 3 from base point 6, the outermost peripheral part of sample 1 was cut with a razor blade so that the sample of the inner layer was not damaged and thus a cut line 8 was provided (see Fig. 4).

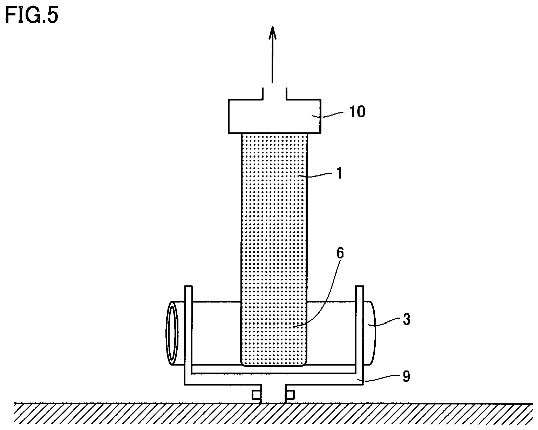

The curved surface sliding stress was measured between the outermost layer part in sample 1, and the inner layer part wound on pipe roll 3 beneath the same (in inner layer). For this measurement, a tensile tester ("Autograph" available from SHIMADZU CORPORATION) was used. Winding core 3 was fixed to a jig 9 placed on the chuck pedestal on the stationary side of the tensile tester (see Fig. 5), and an end part of sample 1 (the end part where alligator clip 4 had been attached) was gripped with a chuck 10 on the load cell side, and stretched at a tension speed of 200 mm/min., and a measurement (tension strength) when sample 1 came off (separated) at cut line 8 was determined as a curved surface sliding stress (except the broken end part, N/50 mm). When the curved surface sliding stress is so strong that it exceeds the breaking strength, and the nonwoven fabric is broken before the sample 1 comes off, the result is indicated by "broken" in Table 1.

In measurement of the curved surface sliding stress, in the condition as shown in Fig. 3(b), sample 1 wound around winding core 3 was pulled out together with alligator clip 4 and weight 5 and broken with a hand, and only one sheet of sample 1 positioned in an inner layer by one lap at the broken part was cut with a razor blade. Winding core 3 was fixed to jig 9 placed on the chuck pedestal on the stationary side of the tensile tester (see Fig. 5), and the end part not being the broken part of sample 1 was gripped with chuck 10 on the load cell side, and stretched at a tension speed of 200 mm/min., and a tension strength when sample 1 came off (separated) was measured, and this measurement was determined as curved surface sliding stress of the broken end part (N/50 mm).

<Example 1>

As a potential crimping fiber, a side-by-side type composite staple fiber ["SOFIT PN-780" available from KURARAY CO., LTD., 1.7 dtex × 51 mm long, machine crimp number: 12 crimps/25 mm, number of crimps after heating at 130°C for 1 minute: 62 crimps/25 mm) composed of a polyethylene terephthalate resin having an intrinsic viscosity of 0.65 [component (A)], and modified polyethylene terephthalate resin [component (B)] in which 20 mol% of isophthalic acid and 5 mol% of diethylene glycol are copolymerized, was prepared. Using 100% by mass of this side-by-side type composite staple fiber, a carded web having a mass per unit area 30 g/m$^2$ was provided by a carding method.

This carded web was moved on the conveyer belt, and allowed to pass between the conveyer belt and a porous plate drum having pores (circular) with a diameter of 2 mm$\phi$ arranged at a pitch of 2 mm in a hound's tooth check, and from inside the porous plate drum, a water flow was injected in a spray form at 0.8 MPa toward the web and the conveyer net, and thus a localizing step for periodically forming a low-density region and a high-density region of fibers was conducted.

Then the carded web was transferred to the heating step while the web was overfed at about 200% so that contraction in the next heating step by the water vapor was not interfered.

Then, the carded web was introduced into the water-vapor injecting device provided in the belt conveyer, and water vapor at 0.5 MPa was ejected to the carded web perpendicularly from the water-vapor injecting device to conduct a water vapor treatment to cause emergence of coiled crimps of the potential crimped fibers, and cause intermingling of fibers, and thus a nonwoven fabric was obtained. This water-vapor injecting device was provided in such a manner that a nozzle was installed in one conveyer so as to spray water vapor toward the carded web via the conveyer belt. The pore diameter of the water vapor injecting nozzle was 0.3 mm, and a device in which this nozzle was arranged in one line at a pitch of 2 mm in the widthwise direction of the conveyer was used. The processing speed was 8.5 m/min., and the distance between the nozzle and the conveyer belt of the suction side was 7.5 mm.

Observation of the surface and the section in the thickness direction of the obtained nonwoven fabric under an electron microscope (100-power) revealed that fibers were oriented substantially parallel with the plane direction of the nonwoven fabric, and crimped substantially uniformly in the thickness direction.

<Example 2 (not according to the invention)>

Using 100% by mass of polyethylene terephthalate fiber ("Tetoron" available from TORAY INDUSTRIES, INC., 1.6 dtex × 51 mm long, machine crimp number 15 crimps/25 mm), a carded web having a mass per unit area of about 10 g/m$^2$ was prepared by a carding method. Six sheets of this carded web were overlapped while they were cross-laid, and fibers were intermingled by a needle punching method, to obtain a nonwoven fabric. The working speed was 2.5 m/min., and the needle density (punching number) was a total of 1000 times/cm$^2$ from both sides.

<Example 3>

A nonwoven fabric was obtained in the same manner as in Example 1 except that the carded web used in Example 1 was introduced into a water-vapor injecting device, the injecting pressure of the water vapor was 0.8 MPa, the working speed was 5.0 m/min, and the distance between the nozzle and the conveyer belt of the suction side was 5.5 mm. Observation of the surface and the section in the thickness direction of the obtained nonwoven fabric under an electron microscope (100-power) revealed that fibers were oriented substantially parallel with the plane direction of the nonwoven fabric, and crimped substantially uniformly in the thickness direction.

<Comparative example 1>

While the carded web used in Example 2 (not according to the invention) was transferred to the belt conveyer equipped with an endless belt formed of resin having 76 mesh and a width of 500 mm, water was injected from nozzles by using a nozzle in which orifices with a diameter of 0.1 mm are aligned at an interval of 0.6 mm in the widthwise direction of the web, in two stages for each of the front and the back sides, and the fibers were intermingled at a working speed of 30 m/min to obtain a nonwoven fabric. The injecting water pressure was 3 MPa for both the front and the back sides in the nozzle line of the former stage, and 5 MPa for both the front and the back sides in the nozzle line of the latter stage.

<Comparative example 2>

A nonwoven fabric was obtained in the same manner as in Example 2 except that the needle density (punching number) was a total of 250 times/cm$^2$ from both sides.

<Comparative example 3>

The carded web used in Example 2 was subjected to a hot air treatment at 130°C by using a hot air circulating processor, to cause emergence of coiled crimps of the potential crimped fibers, and cause intermingling of fibers, and thus a nonwoven fabric was obtained.

[Table 1]

| | | Example 1 | Example 2 (not according to the invention) | Example 3 | Comparative Example 1 | Comparative Example 2 | Comparative Example 3 |
|---|---|---|---|---|---|---|---|
| Mean number of coiled crimps | (number/mm) | 8.1 | - | 32.5 | - | - | 33.5 |
| Mean crimping pitch | ($\mu$m) | 124 | - | 29 | - | - | 28 |
| Mean radius of curvature | ($\mu$m) | 55 | - | 26 | - | - | 27 |
| Curvature of fiber | Front layer | 2.01 | - | 8.51 | - | - | 7.38 |
| | Internal layer | 1.96 | - | 7.31 | - | - | 6.53 |
| | Back layer | 2.43 | - | 7.58 | - | - | 8.25 |
| | Uniformity (%) | 80.6 | - | 85.9 | - | - | 79.1 |
| Mass per unit area | (g/m$^2$) | 89.0 | 144.5 | 135.2 | 35.0 | 68.2 | 130.5 |
| Thickness | (mm) | 1.14 | 0.85 | 1.12 | 0.83 | 1.30 | 1.05 |
| Density | (g/cm$^3$) | 0.08 | 0.17 | 0.12 | 0.04 | 0.05 | 0.12 |
| Breaking strength | MD (N/50mm) | 15.3 | 19.4 | 23.6 | 106.7 | 6.0 | 19.0 |
| | CD (N/50mm) | 2.8 | 29.3 | 6.0 | 29.5 | 9.1 | 5.0 |
| Breaking elongation | MD (%) | 96 | 83 | 245 | 61 | 118 | 231 |
| | CD (%) | 116 | 198 | 153 | 182 | 36 | 166 |
| Stress at 50% extension | MD (N/50mm) | 6.2 | 8.5 | 6.0 | 81.4 | 2.6 | 4.5 |
| | CD (N/50mm) | 1.1 | 12.9 | 1.5 | 22.5 | 4.0 | 1.2 |
| Recovery rate after 50% extension | MD (%) | 93.4 | 53.3 | 92.6 | 59.5 | 67.5 | 93.6 |
| | CD (%) | 93.2 | 47.2 | 90.5 | 56.6 | 62.5 | 88.6 |
| Compressive stress | (kPa) | 1.12 | 2.62 | 2.86 | 0.32 | 0.15 | 4.85 |
| Broken end part length D | (mm) | 16 | 35 | 27 | 88 | 63 | 92 |
| Curved surface sliding stress (except broken end part, N/50 mm) | | Broken | 8.9 | Broken | 0.4 | 2.3 | Broken |
| Curved surface stress of broken end part (N/50 mm) | | 13.7 | 8.2 | 18.9 | 0 | 0.2 | 3.6 |

[Table 2]

| | | | Example 1 | Example 2 (not according to the invention) | Example 3 | Comparative Example 1 | Comparative Example 2 | Comparative Example 3 |
|---|---|---|---|---|---|---|---|---|
| Nonwoven fabric having overall width of 5 cm | Rectangular area 1 | Maximum value | 1.09 | 1.03 | 1.06 | 1.15 | 1.18 | 1.12 |
| | | Minimum value | 0.95 | 0.98 | 0.94 | 0.94 | 0.88 | 0.93 |
| | | Evaluation | A | A | A | B | B | B |
| | Rectangular area 2 | Maximum value | 1.07 | 1.06 | 1.04 | 1.13 | 1.16 | 1.23 |
| | | Minimum value | 0.94 | 0.95 | 0.91 | 0.85 | 0.79 | 0.80 |
| | | Evaluation | A | A | A | B | B | B |
| | Rectangular area 3 | Maximum value | 1.06 | 1.03 | 1.08 | 1.10 | 1.11 | 1.11 |
| | | Minimum value | 0.96 | 0.91 | 0.92 | 0.88 | 0.84 | 0.87 |
| | | Evaluation | A | A | A | B | B | B |

(continued)

| Mass per unit area ratio $W_i/W_{i+1}$ | Nonwoven fabric having overall width of 10 cm | | | Example 1 | Example 2 (not according to the invention) | Example 3 | Comparative Example 1 | Comparative Example 2 | Comparative Example 3 |
|---|---|---|---|---|---|---|---|---|---|
| | | Rectangular area 1 | Maximum value | 1.09 | 1.08 | 1.05 | 1.12 | 1.13 | 1.22 |
| | | | Minimum value | 0.93 | 0.92 | 0.91 | 0.88 | 0.82 | 0.85 |
| | | | Evaluation | A | A | A | B | B | B |
| | | Rectangular area 2 | Maximum value | 1.08 | 1.07 | 1.07 | 1.20 | 1.15 | 1.14 |
| | | | Minimum value | 0.91 | 0.92 | 0.93 | 0.88 | 0.83 | 0.88 |
| | | | Evaluation | A | A | A | B | B | B |
| | | Rectangular area 3 | Maximum value | 1.05 | 1.09 | 1.08 | 1.16 | 1.18 | 1.11 |
| | | | Minimum value | 0.94 | 0.91 | 0.92 | 0.88 | 0.79 | 0.88 |
| | | | Evaluation | A | A | A | B | B | B |

(continued)

| | | | | Example 1 | Example 2 (not according to the invention) | Example 3 | Comparative Example 1 | Comparative Example 2 | Comparative Example 3 |
|---|---|---|---|---|---|---|---|---|---|
| Nonwoven fabric having overall width of 20 cm | | Rectangular area 1 | Maximum value | 1.10 | 1.09 | 1.10 | 1.11 | 1.41 | 1.24 |
| | | | Minimum value | 0.90 | 0.91 | 0.90 | 0.88 | 0.68 | 0.84 |
| | | | Evaluation | A | A | A | B | B | B |
| | | Rectangular area 2 | Maximum value | 1.09 | 1.09 | 1.09 | 1.25 | 1.17 | 1.23 |
| | | | Minimum value | 0.94 | 0.90 | 0.92 | 0.81 | 0.79 | 0.85 |
| | | | Evaluation | A | A | A | B | B | B |
| | | Rectangular area 3 | Maximum value | 1.07 | 1.06 | 1.08 | 1.15 | 1.22 | 1.17 |
| | | | Minimum value | 0.91 | 0.91 | 0.91 | 0.83 | 0.81 | 0.82 |
| | | | Evaluation | A | A | A | B | B | B |

[Table 3]

| Nonwoven fabric having overall width of 5 cm | | | Example 1 | Example 2 (not according to the invention) | Example 3 | Comparative Example 1 | Comparative Example 2 | Comparative Example 3 |
|---|---|---|---|---|---|---|---|---|
| Weight of divisional area (g) | Rectangular area 1 | Divisional area 1 | 0.0442 | 0.0863 | 0.0643 | 0.0209 | 0.0359 | 0.0675 |
| | | Divisional area 2 | 0.0462 | 0.0864 | 0.0648 | 0.0181 | 0.0407 | 0.067 |
| | | Divisional area 3 | 0.043 | 0.0836 | 0.0642 | 0.0193 | 0.0396 | 0.0639 |
| | | Divisional area 4 | 0.0455 | 0.0855 | 0.0606 | 0.0181 | 0.0405 | 0.0571 |
| | | Divisional area 5 | 0.0417 | 0.0827 | 0.0645 | 0.0179 | 0.0343 | 0.0611 |
| | Rectangular area 2 | Divisional area 1 | 0.0441 | 0.0754 | 0.0693 | 0.0171 | 0.0354 | 0.0704 |
| | | Divisional area 2 | 0.0451 | 0.0712 | 0.0682 | 0.0152 | 0.0336 | 0.0754 |
| | | Divisional area 3 | 0.0422 | 0.0712 | 0.0725 | 0.0179 | 0.0335 | 0.0627 |
| | | Divisional area 4 | 0.0417 | 0.0697 | 0.07 | 0.0177 | 0.0288 | 0.0785 |
| | | Divisional area 5 | 0.0445 | 0.0731 | 0.0769 | 0.0159 | 0.0366 | 0.064 |

(continued)

| Nonwoven fabric having overall width of 5 cm | | | Example 1 | Example 2 (not according to the invention) | Example 3 | Comparative Example 1 | Comparative Example 2 | Comparative Example 3 |
|---|---|---|---|---|---|---|---|---|
| | Rectangular area 3 | Divisional area 1 | 0.0451 | 0.0564 | 0.0736 | 0.0178 | 0.0237 | 0.067 |
| | | Divisional area 2 | 0.0436 | 0.0596 | 0.0739 | 0.0179 | 0.0282 | 0.0602 |
| | | Divisional area 3 | 0.0412 | 0.0657 | 0.0804 | 0.0188 | 0.0333 | 0.0693 |
| | | Divisional area 4 | 0.0429 | 0.0637 | 0.0747 | 0.0171 | 0.0301 | 0.0672 |
| | | Divisional area 5 | 0.0423 | 0.0648 | 0.0743 | 0.0195 | 0.0302 | 0.0672 |

[Table 4]

| Nonwoven fabric having overall width of 10 cm | | | Example 1 | Example 2 (not according to the invention) | Example 3 | Comparative Example 1 | Comparative Example 2 | Comparative Example 3 |
|---|---|---|---|---|---|---|---|---|
| Rectangular area 1 | | Divisionalarea 1 | 0.0441 | 0.0717 | 0.0588 | 0.0192 | 0.0339 | 0.0658 |
| | | Divisionalarea 2 | 0.0445 | 0.0731 | 0.0633 | 0.0191 | 0.0369 | 0.0578 |
| | | Divisionalarea 3 | 0.0479 | 0.0692 | 0.0656 | 0.0189 | 0.0327 | 0.0613 |
| | | Divisionalarea 4 | 0.0443 | 0.0643 | 0.0623 | 0.0189 | 0.0304 | 0.0621 |
| | | Divisionalarea 5 | 0.0452 | 0.0644 | 0.0649 | 0.0194 | 0.0306 | 0.0734 |
| | | Divisionalarea 6 | 0.0422 | 0.0698 | 0.071 | 0.0211 | 0.0373 | 0.0601 |
| | | Divisionalarea 7 | 0.0451 | 0.074 | 0.0726 | 0.0189 | 0.0351 | 0.0685 |
| | | Divisionalarea 8 | 0.0449 | 0.0734 | 0.0694 | 0.0201 | 0.0395 | 0.0669 |
| | | Divisionalarea 9 | 0.0413 | 0.0767 | 0.0684 | 0.0192 | 0.0359 | 0.0643 |
| | | Divisionalarea 10 | 0.0423 | 0.0776 | 0.0694 | 0.0217 | 0.0366 | 0.0628 |

| Nonwoven fabric having overall width of 10 cm | | | Example 1 | Example 2 (not according to the invention) | Example 3 | Comparative Example 1 | Comparative Example 2 | Comparative Example 3 |
|---|---|---|---|---|---|---|---|---|
| Weight of divisional area (g) | Rectangular area 2 | Divisional area 1 | 0.0411 | 0.0685 | 0.0647 | 0.0177 | 0.0322 | 0.0612 |
| | | Divisional area 2 | 0.0446 | 0.0728 | 0.0605 | 0.0167 | 0.0342 | 0.0661 |
| | | Divisional area 3 | 0.0419 | 0.0705 | 0.065 | 0.0185 | 0.0331 | 0.0668 |
| | | Divisional area 4 | 0.0405 | 0.0681 | 0.0632 | 0.0194 | 0.0288 | 0.0587 |
| | | Divisional area 5 | 0.0417 | 0.0636 | 0.0673 | 0.0182 | 0.0301 | 0.0555 |
| | | Divisional area 6 | 0.0398 | 0.0668 | 0.0635 | 0.0206 | 0.0362 | 0.063 |
| | | Divisional area 7 | 0.0438 | 0.0727 | 0.0675 | 0.0171 | 0.0388 | 0.0681 |
| | | Divisional area 8 | 0.0407 | 0.0756 | 0.0637 | 0.0178 | 0.0356 | 0.0696 |
| | | Divisional area 9 | 0.0437 | 0.0734 | 0.0682 | 0.0192 | 0.0346 | 0.0678 |
| | | Divisional area 10 | 0.0453 | 0.075 | 0.0686 | 0.0193 | 0.0387 | 0.068 |

(continued)

| Nonwoven fabric having overall width of 10 cm | | Example 1 | Example 2 (not according to the invention) | Example 3 | Comparative Example 1 | Comparative Example 2 | Comparative Example 3 |
|---|---|---|---|---|---|---|---|
| Rectangular area 3 | Divisional area 1 | 0.0408 | 0.065 | 0.067 | 0.0157 | 0.0307 | 0.0683 |
| | Divisional area 2 | 0.0432 | 0.0705 | 0.0658 | 0.0172 | 0.038 | 0.0615 |
| | Divisional area 3 | 0.0412 | 0.0695 | 0.069 | 0.0174 | 0.033 | 0.0696 |
| | Divisional area 4 | 0.0419 | 0.0699 | 0.0676 | 0.0168 | 0.0375 | 0.0685 |
| | Divisional area 5 | 0.0441 | 0.0765 | 0.0628 | 0.0162 | 0.0406 | 0.0683 |
| | Divisional area 6 | 0.044 | 0.072 | 0.0663 | 0.0173 | 0.0344 | 0.0643 |
| | Divisional area 7 | 0.0423 | 0.0659 | 0.0721 | 0.0156 | 0.0307 | 0.0629 |
| | Divisional area 8 | 0.0428 | 0.0611 | 0.0702 | 0.0178 | 0.029 | 0.0663 |
| | Divisional area 9 | 0.0424 | 0.0594 | 0.0696 | 0.0186 | 0.028 | 0.0665 |
| | Divisional area 10 | 0.0451 | 0.0643 | 0.0731 | 0.0161 | 0.0353 | 0.065 |

[Table 5]

| Nonwoven fabric having overall width of 20 cm | | | Example 1 | Example 2 (not according to the invention) | Example 3 | Comparative Example 1 | Comparative Example 2 | Comparative Example 3 |
|---|---|---|---|---|---|---|---|---|
| | Rectangular area 1 | Divisional area 1 | 0.0503 | 0.0654 | 0.0667 | 0.0199 | 0.031 | 0.0585 |
| | | Divisional area 2 | 0.0518 | 0.0677 | 0.0684 | 0.0191 | 0.0458 | 0.0647 |
| | | Divisional area 3 | 0.0484 | 0.0689 | 0.0643 | 0.0188 | 0.0324 | 0.0647 |
| | | Divisional area 4 | 0.0447 | 0.0758 | 0.067 | 0.0183 | 0.0358 | 0.0645 |
| | | Divisional area 5 | 0.0446 | 0.0758 | 0.0674 | 0.0165 | 0.0358 | 0.0608 |
| | | Divisional area 6 | 0.0432 | 0.0718 | 0.0638 | 0.0174 | 0.0338 | 0.0723 |
| | | Divisional area 7 | 0.0454 | 0.0706 | 0.0711 | 0.0174 | 0.0325 | 0.0585 |
| | | Divisional area 8 | 0.0427 | 0.0756 | 0.0672 | 0.018 | 0.0374 | 0.0577 |
| | | Divisional area 9 | 0.0454 | 0.0722 | 0.0618 | 0.0167 | 0.0342 | 0.0622 |
| | | Divisional area 10 | 0.0433 | 0.0725 | 0.0622 | 0.0159 | 0.039 | 0.0613 |
| | | Divisional area 11 | 0.0421 | 0.0706 | 0.0685 | 0.016 | 0.0284 | 0.0641 |
| | | Divisional area 12 | 0.0428 | 0.0702 | 0.0656 | 0.0166 | 0.0334 | 0.068 |
| | | Divisional area 13 | 0.0464 | 0.0698 | 0.062 | 0.0158 | 0.0376 | 0.0587 |
| | | Divisional area 14 | 0.042 | 0.0747 | 0.0626 | 0.0165 | 0.0306 | 0.0621 |

(continued)

| Nonwoven fabric having overall width of 20 cm | Example 1 | Example 2 (not according to the invention) | Example 3 | Comparative Example 1 | Comparative Example 2 | Comparative Example 3 |
|---|---|---|---|---|---|---|
| Divisional area 15 | 0.0429 | 0.0788 | 0.0622 | 0.0179 | 0.0418 | 0.069 |
| Divisional area 16 | 0.0477 | 0.0826 | 0.0675 | 0.0164 | 0.0387 | 0.0648 |
| Divisional area 17 | 0.0465 | 0.079 | 0.0626 | 0.0151 | 0.0424 | 0.0634 |
| Divisional area 18 | 0.0477 | 0.0738 | 0.068 | 0.0171 | 0.0348 | 0.0699 |
| Divisional area 19 | 0.0433 | 0.0677 | 0.0676 | 0.0173 | 0.0319 | 0.0639 |
| Divisional area 20 | 0.0435 | 0.0656 | 0.0612 | 0.0166 | 0.0451 | 0.0602 |
| Divisional area 1 | 0.0483 | 0.0716 | 0.0674 | 0.0145 | 0.0339 | 0.061 |
| Divisional area 2 | 0.0482 | 0.0688 | 0.0673 | 0.0162 | 0.0321 | 0.0649 |
| Divisional area 3 | 0.0507 | 0.071 | 0.073 | 0.0178 | 0.0335 | 0.0603 |
| Divisional area 4 | 0.0502 | 0.072292 | 0.0693 | 0.0153 | 0.0341 | 0.0654 |
| Divisional area 5 | 0.0478 | 0.067 | 0.0713 | 0.0154 | 0.0317 | 0.0671 |
| Divisional area 6 | 0.044 | 0.0672 | 0.0715 | 0.0161 | 0.0319 | 0.0754 |
| Divisional area 7 | 0.0458 | 0.0676 | 0.0683 | 0.0198 | 0.0339 | 0.0612 |
| Divisional area 8 | 0.0443 | 0.0618 | 0.0738 | 0.0159 | 0.0289 | 0.0717 |

(continued)

| Nonwoven fabric having overall width of 20 cm | | | Example 1 | Example 2 (not according to the invention) | Example 3 | Comparative Example 1 | Comparative Example 2 | Comparative Example 3 |
|---|---|---|---|---|---|---|---|---|
| Weight of divisional area (g) | Rectangular area 2 | Divisional area 9 | 0.0444 | 0.0602 | 0.0715 | 0.0142 | 0.0284 | 0.0719 |
| | | Divisional area 10 | 0.045 | 0.0652 | 0.0707 | 0.0172 | 0.0311 | 0.0632 |
| | | Divisional area 11 | 0.0432 | 0.0689 | 0.0649 | 0.0173 | 0.0324 | 0.0726 |
| | | Divisional area 12 | 0.0421 | 0.0766 | 0.0637 | 0.0155 | 0.0362 | 0.0631 |
| | | Divisional area 13 | 0.0421 | 0.0734 | 0.0637 | 0.0168 | 0.0348 | 0.0671 |
| | | Divisional area 14 | 0.0436 | 0.0727 | 0.0666 | 0.0184 | 0.0343 | 0.0688 |
| | | Divisional area 15 | 0.0463 | 0.0687 | 0.066 | 0.0183 | 0.0325 | 0.0657 |
| | | Divisional area 16 | 0.0424 | 0.0729 | 0.0664 | 0.0195 | 0.0344 | 0.0583 |
| | | Divisional area 17 | 0.0442 | 0.0809 | 0.0687 | 0.0193 | 0.0437 | 0.0549 |
| | | Divisional area 18 | 0.0411 | 0.0856 | 0.0716 | 0.0171 | 0.0404 | 0.0599 |
| | | Divisional area 19 | 0.0411 | 0.0856 | 0.0731 | 0.0178 | 0.0401 | 0.0643 |
| | | Divisional area 20 | 0.0419 | 0.084 | 0.0756 | 0.0173 | 0.035 | 0.0613 |
| | | Divisional area 1 | 0.0437 | 0.0742 | 0.068 | 0.0197 | 0.035 | 0.0691 |
| | | Divisional area 2 | 0.0479 | 0.0762 | 0.0724 | 0.0187 | 0.0362 | 0.0603 |

EP 3 239 377 B1

| Nonwoven fabric having overall width of 20 cm | | | Example 1 | Example 2 (not according to the invention) | Example 3 | Comparative Example 1 | Comparative Example 2 | Comparative Example 3 |
|---|---|---|---|---|---|---|---|---|
| Rectangular area 3 | | Divisionalarea 3 | 0.0506 | 0.0724 | 0.0685 | 0.0193 | 0.0341 | 0.0717 |
| | | Divisionalarea 4 | 0.0524 | 0.0722 | 0.0716 | 0.0178 | 0.0341 | 00614 |
| | | Divisionalarea 5 | 0.0523 | 0.0692 | 0.0713 | 0.0182 | 0.0279 | 0.0644 |
| | | Divisionalarea 6 | 0.0489 | 0.0661 | 0.0784 | 0.0169 | 0.0315 | 0.065 |
| | | Divisionalarea 7 | 0.046 | 0.0669 | 0.0727 | 0.0203 | 0.0335 | 0.0679 |
| | | Divisionalarea 8 | 0.0453 | 0.063 | 0.0749 | 0.0176 | 0.0301 | 0.0701 |
| | | Divisionalarea 9 | 0.0471 | 0.0658 | 0.0692 | 0.0166 | 0.0308 | 0.0702 |
| | | Divisionalarea 10 | 0.045 | 0.0642 | 0.0667 | 0.0173 | 0.0302 | 0.0611 |
| | | Divisionalarea 11 | 0.0473 | 0.066 | 0.0701 | 0.0189 | 0.0362 | 0.0585 |
| | | Divisionalarea 12 | 0.0454 | 0.0657 | 0.0687 | 0.0184 | 0.031 | 0.0658 |
| | | Divisionalarea 13 | 0.0466 | 0.0667 | 0.0708 | 0.0169 | 0.0293 | 0.0626 |
| | | Divisionalarea 14 | 0.0481 | 0.0731 | 0.0763 | 0.0189 | 0.036 | 0.0652 |
| | | Divisionalarea 15 | 0.047 | 0.0743 | 0.0726 | 0.0181 | 0.035 | 0.0715 |
| | | Divisionalarea 16 | 0.0501 | 0.0713 | 0.0731 | 0.01823 | 0.0321 | 0.0632 |

EP 3 239 377 B1

(continued)

| Nonwoven fabric having overall width of 20 cm | | Example 1 | Example 2 (not according to the invention) | Example 3 | Comparative Example 1 | Comparative Example 2 | Comparative Example 3 |
|---|---|---|---|---|---|---|---|
| | Divisional area 17 | 0.0488 | 0.077 | 0.0674 | 0.0183 | 0.0367 | 0.0647 |
| | Divisional area 18 | 0.0461 | 0.0797 | 0.0684 | 0.0174 | 0.0424 | 0.0677 |
| | Divisional area 19 | 0.047 | 0.075 | 0.0733 | 0.0163 | 0.0356 | 0.0591 |
| | Divisional area 20 | 0.0472 | 0.0814 | 0.0699 | 0.0157 | 0.0383 | 0.072 |

REFERENCE SIGNS LIST

1: Sample, 2: One-side adhesive tape, 3: Winding core, 4: Alligator clip, 5: Weight, 6: Base point, 7: Point of a half lap from base point, 8: Cut line, 9: Jig, 10: Chuck, 100: Nonwoven fabric, 200: Rectangular area

**Claims**

1.  A nonwoven fabric, wherein in a broken end part formed by a tensile test in accordance with JIS L 1913 of stretching in one direction to lead breakage, letting an innermost point in said one direction be $P_{in}$ and an outermost point be $P_{out}$, distance D along said one direction between point $P_{in}$ and point $P_{out}$ is greater than or equal to 0.1 mm and less than or equal to 50 mm,

    having a lengthwise direction and a widthwise direction, wherein said one direction is parallel with said lengthwise direction, and
    containing a rectangular area having a length of 5 cm in the lengthwise direction and a length in the widthwise direction equivalent to the overall width of said nonwoven fabric,
    wherein in two or more divisional areas contained in said rectangular area each having a length in the lengthwise direction of 5 cm and a length in the widthwise direction of 1 cm, a ratio $W_i/W_{i+1}$ between a mass per unit area $W_i$ of an i-th divisional area from one end of the widthwise direction of said nonwoven fabric (provided that i is an integer of greater than or equal to 1, and a first divisional area contains said one end of the widthwise direction) and a mass per unit area Wi+i of an (i+1)th divisional area is 0.9 to 1.1,
    wherein the nonwoven fabric contains crimped fibers that are crimped in a coiled form,
    wherein the crimped fibers are formed of a composite fiber in which a plurality of resins having different coefficients of thermal contraction form a phase structure,
    wherein the mean fineness of the composite fiber is the range of 0.1 to 50 dtex, wherein the mean fiber length of the composite fiber is 10 to 100 mm,
    wherein the number of crimps before heating (machine crimp number) is 1 to 25 crimps/25 mm, and
    wherein the nonwoven fabric is produced by a method including the step of forming a web from fibers including the composite fiber (web formation step), the step of making fibers in the fiber web be localized in the plane (localizing step), the step of entangling at least part of the fibers in the fiber web (entangling step), and the step of heating the fiber web to crimp the composite fiber (heating step),
    wherein the localizing step is conducted by spraying or injecting low pressure water to the fiber web, the low pressure water is sprayed or injected to the fiber web intermittently or periodically,
    wherein the ejection pressure of the water in the localizing step is 0.1 to 1.5 MPa.

2.  The nonwoven fabric according to claim 1, containing two or more said rectangular areas.

3.  The nonwoven fabric according to claim 1 or 2, wherein said rectangular area contains three or more said divisional areas, and a ratio between the mass per unit area Wi of said i-th divisional area and a mass per unit area of each of other divisional areas is 0.9 to 1.1.

4.  The nonwoven fabric according to any one of claims 1 to 3, having a density of 0.05 to 0.2 g/cm$^3$.

5.  The nonwoven fabric according to any one of claims 1 to 4, having a compressive stress of 0.2 to 10 kPa measured according to the method described herein.

6.  The nonwoven fabric according to claim 1, wherein said crimped fibers are oriented substantially parallel with a plane direction, and are crimped substantially uniformly in a thickness direction at a mean radius of curvature of 20 to 200 $\mu$m.

7.  The nonwoven fabric according to claim 6, wherein a content of said composite fiber in all fibers constituting said nonwoven fabric is greater than or equal to 80% by mass.

8.  The nonwoven fabric according to any one of claims 1 to 7, wherein the nonwoven fabric does not substantially contain an adhesive, and each fiber constituting said nonwoven fabric is not substantially fused.

9.  The nonwoven fabric according to any one of claims 1 to 8, wherein in at least one plane direction, breaking strength is 5 to 30 N/50 mm, breaking elongation is greater than or equal to 50%, and recovery rate after 50% extension is greater than or equal to 80%.

10. The nonwoven fabric according to any one of claims 1 to 9, wherein a curved surface sliding stress in said broken end part is greater than or equal to 5 N/50 mm measured according to the method described herein.

11. The nonwoven fabric according to any one of claims 1 to 10, wherein a ratio between a breaking strength in the lengthwise direction and a breaking strength in the widthwise direction is 1.5 to 50.

12. The nonwoven fabric according to any one of claims 1 to 11, wherein in a cross section in a thickness direction, each region divided into three parts in the thickness direction has a curvature of fiber of greater than or equal to 1.3, and a ratio between a maximum value and a minimum value of curvature of fiber (minimum value/maximum value) for each of the three regions is greater than or equal to 75%.

13. The nonwoven fabric according to any one of claims 1 to 12, which is a bandage.


**Patentansprüche**

1. Faservliesstoff, wobei in einem Bruchendteil, der durch einen Zugversuch gemäß JIS L 1913 zum Strecken in einer Richtung, um zum Bruch zu führen, gebildet wurde, wenn ein innerster Punkt in der einen Richtung als $P_{in}$ bezeichnet wird und ein äußerster Punkt als $P_{out}$ bezeichnet wird, ein Abstand D entlang der einen Richtung zwischen dem Punkt $P_{in}$ und dem Punkt $P_{out}$ größer oder gleich 0,1 mm und kleiner oder gleich 50 mm ist,

   der eine Längenrichtung und eine Breitenrichtung aufweist, wobei die eine Richtung parallel zur Längenrichtung ist, und
   der einen rechteckigen Bereich mit einer Länge von 5 cm in der Längenrichtung und einer Länge in der Breitenrichtung aufweist, die zur Gesamtbreite des Faservliesstoffs äquivalent ist,
   wobei in zwei oder mehr Teilbereichen, die in dem rechteckigen Bereich enthalten sind, die jeweils eine Länge in der Längenrichtung von 5 cm und eine Länge in der Breitenrichtung von 1 cm aufweisen, ein Verhältnis $W_i/W_{i+1}$ zwischen einer flächenbezogene Masse $W_i$ eines i-ten Teilbereichs von einem Ende der Breitenrichtung des Faservliesstoffs (unter der Voraussetzung, dass i eine ganze Zahl größer oder gleich 1 ist, und ein erster Teilbereich das eine Ende der Breitenrichtung enthält) und einer flächenbezogenen Masse $W_{i+1}$ eines (i+1)ten Teilbereichs 0,9 bis 1,1 beträgt,
   wobei der Faservliesstoff gekräuselte Fasern enthält, die in einer gewendelten Form gekräuselt sind,
   wobei die gekräuselten Fasern aus einer Verbundfaser ausgebildet sind, in der mehrere Harze mit unterschiedlichen Wärmeschrumpfungskoeffizienten eine Phasenstruktur bilden,
   wobei die mittlere Feinheit der Verbundfaser im Bereich von 0,1 bis 50 dtex liegt, wobei die mittlere Faserlänge der Verbundfaser 10 bis 100 mm beträgt,
   wobei die Anzahl der Kräuselungen vor dem Erwärmen (Maschinenkräuselungsanzahl) 1 bis 25 Kräuselungen/25 mm beträgt, und
   wobei der Faservliesstoff durch ein Verfahren hergestellt wird, das den Schritt des Bildens einer Stoffbahn aus Fasern, die die Verbundfaser aufweisen (Stoffbahnbildungsschritt), den Schritt des Lokalisierens der Fasern in der Faserstoffbahn in der Ebene (Lokalisierungsschritt), den Schritt des Verwirrens mindestens eines Teils der Fasern in der Faserstoffbahn (Verwirrungsschritt) und den Schritt des Erwärmens der Faserstoffbahn, um die Verbundfaser zu kräuseln (Erwärmungsschritt), aufweist,
   wobei der Lokalisierungsschritt durch Sprühen oder Einspritzen von Niederdruckwasser auf die Faserstoffbahn ausgeführt wird, wobei das Niederdruckwasser auf die Faserstoffbahn intermittierend oder periodisch gesprüht oder eingespritzt wird,
   wobei der Ausstoßdruck des Wassers im Lokalisierungsschritt 0,1 bis 1,5 MPa beträgt.

2. Faservliesstoff nach Anspruch 1, der zwei oder mehr der rechteckigen Bereiche enthält.

3. Faservliesstoff nach Anspruch 1 oder 2, wobei der rechteckige Bereich drei oder mehr der Teilbereiche enthält, und ein Verhältnis zwischen der flächenbezogenen Masse $W_i$ des i-ten Teilbereichs und einer flächenbezogenen Masse jedes der anderen Teilbereiche 0,9 bis 1,1 beträgt.

4. Faservliesstoff nach einem der Ansprüche 1 bis 3, der eine Dichte von 0,05 bis 0,2 g/cm³ aufweist.

**5.** Faservliesstoff nach einem der Ansprüche 1 bis 4, der eine gemäß dem hierin beschriebenen Verfahren gemessene Druckspannung von 0,2 bis 10 kPa aufweist.

**6.** Faservliesstoff nach Anspruch 1, wobei die gekräuselten Fasern im Wesentlichen parallel zu einer Ebenenrichtung ausgerichtet sind und im Wesentlichen einheitlich in einer Dickenrichtung mit einem mittleren Krümmungsradius von 20 bis 200 μm gekräuselt sind.

**7.** Faservliesstoff nach Anspruch 6, wobei ein Gehalt der Verbundfaser in allen Fasern, die den Faservliesstoff bilden, größer oder gleich 80 Masse-% ist.

**8.** Faservliesstoff nach einem der Ansprüche 1 bis 7, wobei der Faservliesstoff im Wesentlichen keinen Klebstoff enthält, und jede Faser, die den Faservliesstoff bildet, im Wesentlichen nicht verschmolzen ist.

**9.** Faservliesstoff nach einem der Ansprüche 1 bis 8, wobei in mindestens einer Ebenenrichtung die Bruchfestigkeit 5 bis 30 N/50 mm beträgt, die Bruchdehnung größer oder gleich 50% ist, und die Erholungsrate nach 50% Dehnung größer oder gleich 80% ist.

**10.** Faservliesstoff nach einem der Ansprüche 1 bis 9, wobei eine gemäß dem hierin beschriebenen Verfahren gemessene Gleitspannung der gekrümmten Oberfläche im Bruchendteil größer oder gleich 5 N/50 mm ist.

**11.** Faservliesstoff nach einem der Ansprüche 1 bis 10, wobei ein Verhältnis zwischen einer Bruchfestigkeit in der Längenrichtung und einer Bruchfestigkeit in der Breitenrichtung 1,5 bis 50 beträgt.

**12.** Faservliesstoff nach einem der Ansprüche 1 bis 11, wobei in einem Querschnitt in einer Dickenrichtung jeder Bereich, der in der Dickenrichtung in drei Teile geteilt ist, eine Krümmung der Faser aufweist, die größer oder gleich 1,3 ist, und ein Verhältnis zwischen einem Maximalwert und einem Minimalwert der Krümmung der Faser (Minimalwert/Maximalwert) für jeden der drei Bereiche größer oder gleich 75% ist.

**13.** Faservliesstoff nach einem der Ansprüche 1 bis 12, der eine Bandage ist.

## Revendications

**1.** Tissu non-tissé, où, dans une partie d'extrémité déchirée formée par un essai de traction conforme à JIS L 1913 avec étirement dans une direction pour entraîner une rupture, laissant un point le plus intérieur dans ladite direction en tant que $P_{in}$ et un point le plus extérieur en tant que $P_{out}$, la distance D dans ladite direction entre le point $P_{in}$ et le point $P_{out}$ est supérieure ou égale à 0,1 mm et inférieure ou égale à 50 mm,

un sens de la longueur et un sens de la largeur étant présentés, où ladite direction étant parallèle au sens de la longueur, et
une surface rectangulaire étant contenue, avec une longueur de 5 cm dans le sens de la longueur et une longueur dans le sens de la largeur équivalente à la largeur totale du tissu non-tissé,
où, dans au moins deux surfaces divisionnaires contenues dans la surface rectangulaire, chacune ayant une longueur de 5 cm dans le sens de la longueur et une longueur de 1 cm dans le sens de la largeur, un rapport $W_i/W_{i+1}$ entre le grammage $W_i$ d'une $i^{ème}$ surface divisionnaire depuis une extrémité dans le sens de la largeur du tissu non-tissé (à la condition que i soit un entier supérieur ou égal à 1, et qu'une première surface divisionnaire contienne ladite extrémité dans le sens de la largeur) et le grammage $W_{i+1}$ d'une $(i+1)^{eme}$ surface divisionnaire est compris entre 0,9 et 1,1,
où ledit tissu non-tissé contient des fibres frisées avec une forme enroulée,
où les fibres frisées sont formées d'une fibre composite où une pluralité de résines présentant différents coefficients de contraction thermique forment une structure de phase, où la finesse moyenne de la fibre composite est comprise entre 0,1 et 50 dtex,
où la longueur moyenne de la fibre composite est comprise entre 10 et 100 mm,
où le nombre de frisures avant chauffage (nombre de frisures machine) est compris entre 1 et 25 frisures/25 mm, et
où ledit tissu non-tissé est produit au moyen d'un procédé comprenant l'étape de formage d'un voile à partir de fibres comprenant la fibre composite (étape de formation de voile), l'étape de production de fibres dans le voile de carde localisé dans le plan (étape de localisation), l'étape d'enchevêtrement d'au moins une partie des fibres dans le voile de carde (étape d'enchevêtrement), et l'étape de chauffage du voile de carde pour friser la fibre composite (étape de chauffage),

où l'étape de localisation est réalisée par pulvérisation ou injection sous basse pression d'eau sur/dans le voile de carde, l'eau sous basse pression étant pulvérisée ou injectée de manière intermittente ou périodique sur/dans le voile de carde,

où la pressure d'éjection de l'eau lors de l'étape de localisation est comprise entre 0,1 et 1,5 MPa.

2.  Tissu non-tissé selon la revendication 1, contenant au moins deux surfaces rectangulaires.

3.  Tissu non-tissé selon la revendication 1 ou la revendication 2, où la surface rectangulaire contient au moins trois surfaces divisionnaires, et le rapport entre le grammage $W_i$ de la $i^{ème}$ surface divisionnaire et le grammage de chacune des autres surfaces divisionnaires est compris entre 0,9 et 1,1.

4.  Tissu non-tissé selon l'une des revendications 1 à 3, ayant une densité comprise entre 0,05 et 0,2 g/cm$^3$.

5.  Tissu non-tissé selon l'une des revendications 1 à 4, ayant une résistance à la compression comprise entre 0,2 et 10 kPa, mesurée conformément au procédé décrit.

6.  Tissu non-tissé selon la revendication 1, où les fibres frisées sont orientées sensiblement parallèlement à une direction du plan, et sont frisées sensiblement uniformément dans le sens de l'épaisseur avec un rayon de courbure moyen compris entre 20 et 200 $\mu$m.

7.  Tissu non-tissé selon la revendication 6, où la teneur de la fibre composite dans toutes les fibres constituant le tissu non-tissé est supérieure ou égale à 80 % en masse.

8.  Tissu non-tissé selon l'une des revendications 1 à 7, où ledit tissu non-tissé ne contient pratiquement aucun adhésif, et où chaque fibre constituant ledit tissu non-tissé n'est pratiquement pas fondue.

9.  Tissu non-tissé selon l'une des revendications 1 à 8, où, dans au moins une direction du plan, la résistance à la rupture est comprise entre 5 et 30 N/50 mm, l'allongement à la rupture est supérieur ou égal à 50 %, et le taux de récupération après une extension de 50 % est supérieur ou égal à 80 %.

10. Tissu non-tissé selon l'une des revendications 1 à 9, où une contrainte de glissement sur surface courbe dans la partie d'extrémité déchirée est supérieure ou égale à 5 N/50 mm, mesurée conformément au procédé décrit.

11. Tissu non-tissé selon l'une des revendications 1 à 10, où le rapport entre la résistance à la rupture dans le sens de la longueur et la résistance à la rupture dans le sens de la largeur est compris entre 1,5 et 50.

12. Tissu non-tissé selon l'une des revendications 1 à 11, où, en section transversale dans le sens de l'épaisseur, chaque zone divisée en trois parties dans le sens de l'épaisseur présente une courbure de fibre supérieure ou égale à 1,3, et où le rapport entre une valeur maximale et une valeur minimale de la courbure de fibre (valeur minimale/valeur maximale) pour chacune des trois zones est supérieur ou égal à 75 %.

13. Tissu non-tissé selon l'une des revendications 1 à 12, lequel est un bandage.

FIG.1

100

$P_{in}$

$P_{out}$

D

BREAKING DIRECTION

FIG.2

FIG.3

(a)

(b)

FIG.4

FIG.5

FIG.6

(a)

L1

(b)

L1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008015972 A
- EP 2058424 A1
- JP 2014037662 A